# EUROPEAN PATENT SPECIFICATION

(11) **EP 0 885 285 B1**
(45) Date of publication and mention of the grant of the patent: **20.10.2004**
(21) Application number: 97908958.8
(22) Date of filing: 04.03.1997
(51) Int. Cl.: C11D 3/386, C12N 9/28

(54) **DETERGENT COMPOSITIONS COMPRISING PROTEASES AND IMPROVED AMYLASES**
WASCHEMITTELZUSAMMENSETZUNGEN MIT PROTEASEN UND VERBESSERTEN AMYLASEN
COMPOSITIONS DE DETERGENCE COMPRENANT DES PROTEASES ET DES AMYLASES SUPERIEURES

(30) Priority: 07.03.1996 WO PCT/US96/03276
(43) Date of publication of application: 23.12.1998
(73) Proprietor: THE PROCTER & GAMBLE COMPANY, Cincinnati, Ohio 45202 (US)
(72) Inventor: BAECK, Andre, Cesar, B-2820 Bonheiden (BE); JONES, Lynda, Anne, Newcastle-upon-Tyne NE3 4ME (GB); OHTANI, Ryohei, Hyogo 663 (JP); PRAMOD, Kakumanu, West Chester, OH 45069 (US); RAI, Saroj, West Chester, OH 45069 (US); SHOWELL, Michael, Stanford, Cincinnati, OH 45231 (US); WARD, Glenn, Gosforth Newcastle-upon-Tyne NE3 5LL (GB)
(74) Representative: Engisch, Gautier
(86) International application number: PCT/US1997/003635
(87) International publication number: WO 1997/032961

(56) References cited:
- EP-A- 0 368 341
- EP-A- 0 670 367
- EP-A- 0 755 999
- WO-A-94/18314
- WO-A-95/10603
- WO-A-95/26397
- WO-A-95/35382
- WO-A-96/23873
- WO-A-97/18287
- WO-A-97/41213
- BIOCHEM. AND BIOPHYS. RES. COMM., vol. 151, no. 1, 29 February 1988, pages 25-31, XP000605386 A. TSUKAMATO ET AL.: "Nucleotide Sequence Of The Maltohexaose-Producing Amylase Gene From An Alkalophilic Bacillus sp. 707 And Structural Similarity To Liquifying Type -Amylases"

## Description

### TECHNICAL FIELD

The present invention relates to detergent compositions comprising certain levels of specific amylase enzymes along with protease, which improve cleaning and stain removal performances in hard surface cleaning, dishwashing and laundry.

### BACKGROUND OF THE INVENTION

For a number of years amylase enzymes have been used for a variety of different purposes, the most important of which are starch liquefaction, textile desizing, starch modification in the paper and pulp industry, and for brewing and baking. A further use of amylases which is becoming increasingly important, is the removal of starch containing soils and stains during the washing of fabrics, hard surfaces and dishes.

Indeed, amylase enzymes have long been recognised in dishwashing, hard surface cleaning and laundry compositions to provide the removal of starchy food residues or starchy films from dishware, flatware, glasses and hard surfaces or to provide cleaning performance on starchy soils as well as other soils typically encountered in laundry applications.

WO/94/02597, Novo Nordisk A/S published February 03, 1994, describes cleaning compositions which incorporate mutant amylases. See also WO/94/18314, Genencor, published August 18, 1994 and WO/95/10603, Novo Nordisk A/S, published April 20,1995.

Other amylases known for use in cleaning compositions include both α- and β-amylases. α-Amylases are known in the art and include those disclosed in US Pat. no. 5,003,257; EP 252,666; WO/91/00353; FR 2,676,456; EP 285,123; EP 525,610; EP 368,341; and British Patent specification no. 1,296,839 (Novo). EP 755 999 A1 describes detergent compositions comprising an oxidative stability - enhand amylase and a protease at a level of 0,0001 % to 5 % pure protease enzyme by weight. WO 97/18287 discloses a per acid based automatic dishwashing detergent composition comprising a bleach resistant amylase.

Recently new amylases have been identified and are described in WO/95/26397, Novo Nordisk A/S, published October 05, 1995, disclosing an α-amylase having a specific activity at least 25% higher than the specific activity of Termamyl® at a temperature range of 25°C to 55°C and at a pH value in the range of 8 to 10. Variants of these new amylases demonstrating at least one of the following properties relative to the parent enzymes : 1) improved thermal stability, 2) oxidation stability and 3) reduced calcium ion dependency properties. Examples of other desirable improvements or modifications of properties (relative to the parent-α-amylase) which may be achieved with a variant according to the present invention are : increased stability and/or α-amylolytic activity at neutral to relatively high pH values, increased α-amylolytic activity at relatively high temperature and increase or decrease of the isoelectric point (pI) so as to better match the pI value for α-amylase variant to the pH of the medium, have been described in the co-pending application by Novo Nordisk WO 96/23873. Such newly developed amylases - hereinafter referred as "specific amylase enzymes" - are generally used in detergent compositions at levels of 0.0001 to 0.1% pure enzyme by weight of the total composition.

It is an object of the present invention to provide detergent compositions containing certain levels expressed in pure enzyme by weight of the total composition, of specific amylase enzymes with enhanced overall cleaning and stain removal performance.

It has now surprisingly been found that optimised applications of such specific amylase enzymes into detergent compositions is obtained when the concentration of the enzyme ranges from 0.00018% to 0.060% pure enzyme by weight of the total composition, more preferably from 0.00024% to 0.048% pure enzyme by weight of the total composition.

It is another object of the present invention to provide detergent compositions containing said levels of specific amylase enzymes together with a protease and optionally other detergent ingredients selected from a complementary amylase, a cationic surfactant, a bleach system, a builder, a chlorine scavenger, a dispersant and/or mixtures thereof.

### SUMMARY OF THE INVENTION

The present invention relates to detergent compositions comprising a protease and a specific amylase enzyme at a level from 0.00018% to 0.060% pure enzyme by weight of the total composition, more preferably from 0.00024% to 0.048% pure enzyme by weight of total weight composition.

### DETAILED DESCRIPTION OF THE INVENTION

### Specific Amylase Enzymes

An essential component of the detergent compositions of the present invention is a specific amylase enzyme. Such specific amylase enzymes include those described in WO95/26397 and in co-pending application by Novo Nordisk WO 96/23873. These enzymes are incorporated into detergent compositions at a level from 0.00018% to 0.060% pure enzyme by weight of the total composition, more preferably from 0.00024% to 0.048% pure enzyme by weight of total weight composition.

Specific amylase enzymes for use in the detergent compositions of the present invention therefore include :
(a) α-amylases characterised by having a specific activity at least 25% higher than the specific activity of Termamyl® at a temperature range of 25°C to 55°C and at a pH value in the range of 8 to 10, measured by the Phadebas® α-amylase activity assay. Such Phadebas® α-amylase activity assay is described at pages 9-10, WO95/26397.
(b) α-amylases according (a) comprising the amino sequence shown in SEQ ID No. 1 or an α-amylase being at least 80% homologous with the amino acid sequence shown in SEQ ID No.1.
(c) α-amylases according (a) comprising the amino sequence shown in SEQ ID No.2 or an α-amylase being at least 80% homologous with the amino acid sequence shown in SEQ ID No.2.
(d) α-amylases according (a) comprising the following amino sequence in the N-terminal : His-His-Asn-Gly-Thr-Asn-Gly-Thr-Met-Met-Gln-Tyr-Phe-Glu-Trp-Tyr-Leu-Pro-Asn-Asp (SEQ ID No.3) or an α-amylase being at least 80% homologous with the amino acid sequence shown (SEQ ID No.3) in the N-terminal.
   A polypeptide is considered to be X% homologous to the parent amylase if a comparison of the respective amino acid sequences, performed via algorithms, such as the one described by Lipman and Pearson in Science 227, 1985, p. 1435, reveals an identity of X%
(e) α-amylases according (a-d) wherein the α-amylase is obtainable from an alkalophilic Bacillus species; and in particular, from any of the strains NCIB 12289, NCIB 12512, NCIB 12513 and DSM 935.
   In the context of the present invention, the term "obtainable from" is intended not only to indicate an amylase produced by a Bacillus strain byt also an amylase encoded by a DNA sequence isolated from such a Bacillus strain and produced in an host organism transformed with said DNA sequence.
(f)α-amylase showing positive immunological cross-reactivity with antibodies raised against an α-amylase having an amino acid sequence corresponding respectively co SEQ ID No.1, ID No.2 or ID No.3.
(g) Variants of the following parent α-amylases which (i) have one of the amino acid sequences shown in SEQ ID No.1, ID No.2 or ID No.4 respectively, or (ii)displays at least 80% homology with one or more of said amino acid sequences, and/or displays immunological cross-reactivity with an antibody raised against an α-amylase having one of said amino acid sequences, and/or is encoded by a DNA sequence wich hybridizes with the same probe as a DNA sequence encoding an α-amylase having one of said amino acid sequence; in which variants :
   1. at least one amino acid residue of said parent α-amylase has been deleted; and/or
   2. at least one amino acid residue of said parent α-amylase has been replaced by a different amino acid residue; and/or
   3. at least one amino acid residue has been inserted relative to said parent α-amylase;
said variant having an α-amylase activity and exhibiting at least one of the following properties relative to said parent α-amylase : increased thermostability, increased stability towards oxidation, reduced Ca ion dependency, increased stability and/or α-amylolytic activity at neutral to relatively high pH values, increased α-amylolytic activity at relatively high temperature and increase or decrease of the isoelectric point (pI) so as to better match the pI value for α-amylase variant to the pH of the medium.
Said variants are described in the co-pending patent application WO 96/23873.

It has been found that optimised applications of such specific amylase enzymes into detergent compositions is obtained when the concentration of the enzyme ranges from 0.00018% to 0.060% pure enzyme by weight of the total composition, more preferably from 0.00024% to 0.048% pure enzyme by weight of the total composition.

### Detergent components

The detergent compositions of the invention may also contain additional detergent components. The precise nature of these additional components, and levels of incorporation thereof will depend on the physical form of the composition, and the nature of the cleaning operation for which it is to be used.

The compositions of the invention may for example, be formulated as hard surface cleaner, hand and machine dishwashing compositions, hand and machine laundry detergent compositions including laundry additive compositions and compositions suitable for use in the soaking and/or pretreatment of stained fabrics, rinse added fabric softener compositions.

When formulated as compositions for use in manual dishwashing methods the compositions of the invention preferably contain a surfactant and preferably other detergent compounds selected from organic polymeric compounds, suds enhancing agents, group II metal ions, solvents, hydrotropes and additional enzymes.

When formulated as compositions suitable for use in a laundry machine washing method, the compositions of the invention preferably contain both a surfactant and a builder compound and additionally one or more detergent components preferably selected from organic polymeric compounds, bleaching agents, additional enzymes, suds suppressors, dispersants, lime-soap dispersants, soil suspension and anti-redeposition agents and corrosion inhibitors. Laundry compositions can also contain softening agents, as additional detergent components.

The compositions of the invention can also be used as detergent additive products. Such additive products are intended to supplement or boost the performance of conventional detergent compositions.

If needed the density of the granular laundry detergent compositions herein ranges from 400 to 1200 g/litre, preferably 600 to 950 g/litre of composition measured at 20°C.

The "compact" form of the granular laundry detergent compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition.
In the compact compositions, the filler salt is present in amounts not exceeding 15% of the total composition, preferably not exceeding 10%, most preferably not exceeding 5% by weight of the composition.
The inorganic filler salts, such as meant in the present compositions are selected from the alkali and alkaline-earth-metal salts of sulphates and chlorides.
A preferred filler salt is sodium sulphate.

Liquid detergent compositions according to the present invention can also be in a "concentrated form", in such case, the liquid detergent compositions according the present invention will contain a lower amount of water, compared to conventional liquid detergents.
Typically the water content of the concentrated liquid detergent is preferably less than 40%, more preferably less than 30%, most preferably less than 20% by weight of the detergent composition.

### Surfactant system

The detergent compositions according to the present invention comprise a surfactant system wherein the surfactant can be selected from nonionic and/or anionic and/or cationic and/or ampholytic and/or zwitterionic and/or semi-polar surfactants.

It has been found that combinations of specific amylase enzyme according to the present invention with a cationic surfactant enhance the overall cleaning and stain removal performance.

The surfactant is typically present at a level of from 0.1% to 60% by weight. More preferred levels of incorporation are 1% to 35% by weight, most preferably from 1% to 20% by weight of laundry and rinse added fabric softener compositions in accord with the invention.

The surfactant is preferably formulated to be compatible with enzyme components present in the composition. In liquid or gel compositions the surfactant is most preferably formulated such that it promotes, or at least does not degrade, the stability of any enzyme in these compositions.

Polyethylene, polypropylene, and polybytylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably from about 8 to about 14 carbon atoms, in either a straight-chain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).
The condensation products of primary and secondary aliphatic alcohols with from about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (the condensation product of C₁₁-C₁₅ linear alcohol with 9 moles ethylene oxide), Tergitol™ 24-L-6 NMW (the condensation product of C₁₂-C₁₄ primary alcohol with 6 moles ethylene oxide with a narrow molecular weight distribytion), both marketed by Union Carbide Corporation; Neodol™ 45-9 (the condensation product of C₁₄-C₁₅ linear alcohol with 9 moles of ethylene oxide), Neodol™ 23-3 (the condensation product of C₁₂-C₁₃ linear alcohol with 3.0 moles of ethylene oxide), Neodol™ 45-7 (the condensation product of C₁₄-C₁₅ linear alcohol with 7 moles of ethylene oxide), Neodol™ 45-5 (the condensation product of C₁₄-C₁₅ linear alcohol with 5 moles of ethylene oxide) marketed by Shell Chemical Company, Kyro™ EOB (the condensation product of C₁₃-C₁₅ alcohol with 9 moles ethylene oxide), marketed by The Procter & Gamble Company, and Genapol LA 030 or 050 (the condensation product of C₁₂-C₁₄ alcohol with 3 or 5 moles of ethylene oxide) marketed by Hoechst. Preferred range of HLB in these products is from 8-11 and most preferred from 8-10.

Also useful as the nonionic surfactant of the surfactant systems of the present invention are the alkylpolysaccharides disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g. a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6-positions on the preceding saccharide units.
The preferred alkylpolyglycosides have the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominately the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight of from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially-available Pluronic™ surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic™ compounds, marketed by BASF.

Preferred for use as the nonionic surfactant of the surfactant systems of the present invention are polyethylene oxide condensates of alkyl phenols, condensation products of primary and secondary aliphatic alcohols with from about 1 to about 25 moles of ethylene oxide, alkylpolysaccharides, and mixtures thereof. Most preferred are C₈-C₁₄ alkyl phenol ethoxylates having from 3 to 15 ethoxy groups and C₈-C₁₈ alcohol ethoxylates (preferably C₁₀ avg.) having from 2 to 10 ethoxy groups, and mixtures thereof.

Highly preferred nonionic surfactants are polyhydroxy fatty acid amide surfactants of the formula. 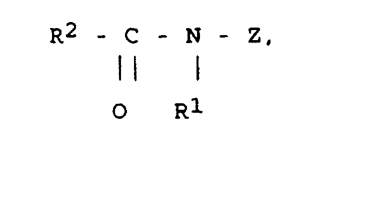 wherein R¹ is H, or R¹ is C₁₋₄ hydrocarbyl, 2-hydroxy ethyl, 2-hydroxy propyl or a mixture thereof, R² is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R¹ is methyl, R² is a straight C₁₁₋₁₅ alkyl or C₁₆₋₁₈ alkyl or alkenyl chain such as coconut alkyl or mixtures thereof, and Z is derived from a reducing sugar such as glucose, fructose, maltose, lactose, in a reductive amination reaction.

When included in such laundry detergent compositions, the nonionic surfactant systems of the present invention act to improve the greasy/oily stain removal properties of such laundry detergent compositions across a broad range of laundry conditions.

Highly preferred anionic surfactants include alkyl alkoxylated sulfate surfactants hereof are water soluble salts or acids of the formula RO(A)ₘSO3M wherein R is an unsubstituted C₁₀-C₂₄ alkyl or hydroxyalkyl group having a C₁₀-C₂₄ alkyl component, preferably a C₁₂-C₂₀ alkyl or hydroxyalkyl, more preferably C₁₂-C₁₈ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are C₁₂-C₁₈ alkyl polyethoxylate (1.0) sulfate (C₁₂-C₁₈E(1.0)M), C₁₂-C₁₈ alkyl polyethoxylate (2.25) sulfate (C₁₂-C₁₈E(2.25)M), C₁₂-C₁₈ alkyl polyethoxylate (3.0) sulfate (C₁₂-C₁₈E(3.0)M), and C₁₂-C₁₈ alkyl polyethoxylate (4.0) sulfate (C₁₂-C₁₈E(4.0)M), wherein M is conveniently selected from sodium and potassium.

Quaternary ammonium surfactant suitable for the present invention has the formula (I): 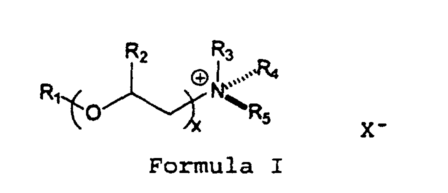 whereby R1 is a short chainlength alkyl (C6-C10) or alkylamidoalkyl of the formula (II) : 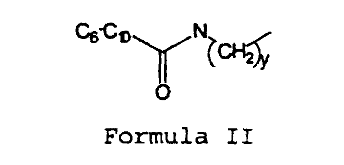 y is 2-4, preferably 3.
whereby R2 is H or a C1-C3 alkyl,
whereby x is 0-4, preferably 0-2, most preferably 0,
whereby R3, R4 and R5 are either the same or different and can be either a short chain alkyl (C1-C3) or alkoxylated alkyl of the formula III,
whereby X- is a counterion, preferably a halide, e.g. chloride or methylsulfate. 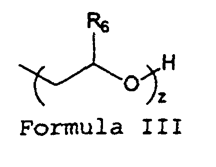 R6 is C₁-C₄ and z is 1 or 2.

Preferred quat ammonium surfactants are those as defined in formula I whereby
R₁ is C₈, C₁₀ or mixtures thereof, x=o,
R₃, R₄ = CH₃ and R₅ = CH₂CH₂OH.

Suitable anionic surfactants to be used are alkyl ester sulfonate surfactants including linear esters of C₈-C₂₀ carboxylic acids (i.e., fatty acids) which are sulfonated with gaseous SO₃ according to "The Journal of the American Oil Chemists Society", 52 (1975), pp. 323-329. Suitable starting materials would include natural fatty substances as derived from tallow, palm oil, etc.
The preferred alkyl ester sulfonate surfactant, especially for laundry applications, comprise alkyl ester sulfonate surfactants of the structural formula : 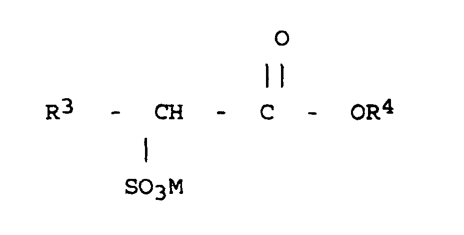 wherein R³ is a C₈-C₂₀ hydrocarbyl, preferably an alkyl, or combination thereof, R⁴ is a C₁-C₆ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethanolamine, and triethanolamine. Preferably, R³ is C₁₀-C₁₆ alkyl, and R⁴ is methyl, ethyl or isopropyl. Especially preferred are the methyl ester sulfonates wherein R³ is C₁₀-C₁₆ alkyl.

Other suitable anionic surfactants include the alkyl sulfate surfactants which are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of C₁₂-C₁₆ are preferred for lower wash temperatures (e.g. below about 50°C) and C₁₆₋₁₈ alkyl chains are preferred for higher wash temperatures (e.g. above about 50°C).

Other anionic surfactants useful for detersive purposes can also be included in the laundry detergent compositions of the present invention. These can include salts (including, for example, sodium, potassium, ammonium, and substituted ammonium salts such as mono-, di- and triethanolamine salts) of soap, C₈-C₂₂ primary of secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated C₁₂-C₁₈ monoesters) and diesters of sulfosuccinates (especially saturated and unsaturated C₆-C₁₂ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula RO(CH₂CH₂O)ₖ-CH₂COO-M+ wherein R is a C₈-C₂₂ alkyl, k is an integer from 1 to 10, and M is a soluble salt-forming cation. Resin acids and hydrogenated resin acids are also suitable, such as rosin, hydrogenated rosin, and resin acids and hydrogenated resin acids present in or derived from tall oil.
Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23 (herein incorporated by reference).
When included therein, the laundry detergent compositions of the present invention typically comprise from about 1% to about 40%, preferably from about 3% to about 20% by weight of such anionic surfactants.

The detergent composition of the present invention may further comprise a cosurfactant selected from the group of primary or tertiary amines.
Suitable primary amines for use herein include amines according to the formula R₁NH₂ wherein R₁ is a C₆-C₁₂, preferably C₆-C₁₀ alkyl chain or R₄X(CH₂)ₙ, X is -O-, -C(O)NH- or -NH-, R₄ is a C₆-C₁₂ alkyl chain n is between 1 to 5, preferably 3. R₁ alkyl chains may be straight or branched and may be interrupted with up to 12, preferably less than 5 ethylene oxide moieties.
Preferred amines according to the formula herein above are n-alkyl amines. Suitable amines for use herein may be selected from 1-hexylamine, 1-octylamine, 1-decylamine and laurylamine. Other preferred primary amines include C8-C10 oxypropylamine, octyloxypropylamine, 2-ethylhexyloxypropylamine, lauryl amido propylamine and amido propylamine.

Suitable tertiary amines for use herein include tertiary amines having the formula R₁R₂R₃N wherein R1 and R2 are C₁-C₈ alkylchains or 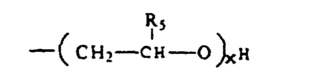 R₃ is either a C₆-C₁₂, preferably C₆-C₁₀ alkyl chain, or R₃ is R₄X(CH₂)ₙ, whereby X is -O-, -C(O)NH- or -NH-,R₄ is a C₄-C₁₂, n is between 1 to 5, preferably 2-3. R₅ is H or C₁-C₂ alkyl and x is between 1 to 6 .
R₃ and R₄ may be linear or branched ; R₃ alkyl chains may be interrupted with up to 12, preferably less than 5, ethylene oxide moieties.

Preferred tertiary amines are R₁R₂R₃N where R1 is a C6-C12 alkyl chain, R2 and R3 are C1-C3 alkyl or 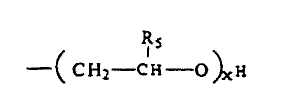 where R5 is H or CH3 and x = 1-2.

Also preferred are the amidoamines of the formula: 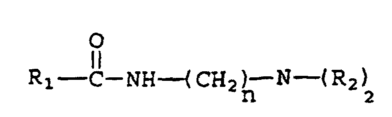 wherein R₁ is C₆-C₁₂ alkyl; n is 2-4,
preferably n is 3; R₂ and R₃ is C₁-C₄

Most preferred amines of the present invention include 1-octylamine, 1-hexylamine, 1-decylamine, 1-dodecylamine,C8-10oxypropylamine, N coco 1-3diaminopropane, coconutalkyldimethylamine, lauryldimethylamine, lauryl bis(hydroxyethyl)amine, coco bis(hydroxyehtyl)amine, lauryl amine 2 moles propoxylated, octyl amine 2 moles propoxylated, lauryl amidopropyldimethylamine, C8-10 amidopropyldimethylamine and C10 amidopropyldimethylamine. The most preferred amines for use in the compositions herein are 1-hexylamine, 1-octylamine, 1-decylamine, 1-dodecylamine. Especially desirable are n-dodecyldimethylamine and bishydroxyethylcoconutalkylamine and oleylamine 7 times ethoxylated, lauryl amido propylamine and cocoamido propylamine.

The laundry detergent compositions of the present invention may also contain cationic, ampholytic, zwitterionic, and semi-polar surfactants, as well as the nonionic and/or anionic surfactants other than those already described herein.
Cationic detersive surfactants suitable for use in the laundry detergent compositions of the present invention are those having one long-chain hydrocarbyl group. Examples of such cationic surfactants include the ammonium surfactants such as alkyltrimethylammonium halogenides, and those surfactants having the formula :

[R²(OR³)_{y}][R⁴(OR³)_{y}]₂R⁵N+X-

wherein R² is an alkyl or alkyl benzyl group having from about 8 to about 18 carbon atoms in the alkyl chain, each R³ is selected from the group consisting of -CH₂CH₂-, -CH₂CH(CH₃)-, -CH₂CH(CH₂OH)-, -CH₂CH₂CH₂-, and mixtures thereof; each R⁴ is selected from the group consisting of C₁-C₄ alkyl, C₁-C₄ hydroxyalkyl, benzyl ring structures formed by joining the two R⁴ groups, -CH₂CHOH-CHOHCOR⁶CHOHCH₂OH wherein R⁶ is any hexose or hexose polymer having a molecular weight less than about 1000, and hydrogen when y is not 0; R⁵ is the same as R⁴ or is an alkyl chain wherein the total number of carbon atoms of R² plus R⁵ is not more than about 18; each y is from 0 to about 10 and the sum of the y values is from 0 to about 15; and X is any compatible anion.

Highly preferred cationic surfactants are the water-soluble quaternary ammonium compounds useful in the present composition having the formula :

R₁R₂R₃R₄N⁺X⁻ (i)

wherein R₁ is C₈-C₁₆ alkyl, each of R₂, R₃ and R₄ is independently C₁-C₄ alkyl, C₁-C₄ hydroxy alkyl, benzyl, and - (C₂H₄₀)ₓH where x has a value from 2 to 5, and X is an anion. Not more than one of R₂, R₃ or R₄ should be benzyl.
The preferred alkyl chain length for R₁ is C₁₂-C₁₅ particularly where the alkyl group is a mixture of chain lengths derived from coconut or palm kernel fat or is derived synthetically by olefin byild up or OXO alcohols synthesis. Preferred groups for R₂R₃ and R₄ are methyl and hydroxyethyl groups and the anion X may be selected from halide, methosulphate, acetate and phosphate ions.
Examples of suitable quaternary ammonium compounds of formulae (i) for use herein are :
coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
C₁₂₋₁₅ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)₄ ammonium chloride or bromide;
choline esters (compounds of formula (i) wherein R₁ is 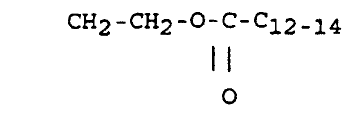 alkyl and R₂R₃R₄ are methyl).
di-alkyl imidazolines (compounds of formula (i)].

Other cationic surfactants useful herein are also described in U.S. Patent 4,228,044, Cambre, issued October 14, 1980 and in European Patent Application EP 000,224.

When included therein, the laundry detergent compositions of the present invention typically comprise from 0.2% to about 25%, preferably from about 1% to about 8% by weight of such cationic surfactants.

Ampholytic surfactants are also suitable for use in the laundry detergent compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate. See U.S. Patent No. 3,929,678 to Laughlin et al., issued December 30, 1975 at column 19, lines 18-35, for examples of ampholytic surfactants.
When included therein, the laundry detergent compositions of the present invention typically comprise from 0.2% to about 15%, preferably from about 1% to about 10% by weight of such ampholytic surfactants.

Zwitterionic surfactants are also suitable for use in laundry detergent compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent No. 3,929,678 to Laughlin et al., issued December 30, 1975 at column 19, line 38 through column 22, line 48, for examples of zwitterionic surfactants.

When included therein, the laundry detergent compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; water-soluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about 1 to about 3 carbon atoms.

Semi-polar nonionic detergent surfactants include the amine oxide surfactants having the formula 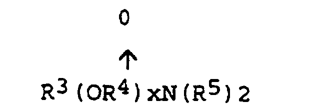 wherein R³ is an alkyl, hydroxyalkyl, or alkyl phenyl group or mixtures therof containing from about 8 to about 22 carbon atoms; R⁴ is an alkylene or hydroxyalkylene group containing from about 2 to about 3 carbon atoms or mixtures thereof; x is from 0 to about 3; and each R⁵ is an alkyl or hydroxyalkyl group containing from about 1 to about 3 carbon atoms or a polyethylene oxide group containing from about 1 to about 3 ethylene oxide groups. The R⁵ groups can be attached to each other, e.g., through an oxygen or nitrogen atom, to form a ring structure.

These amine oxide surfactants in particular include C₁₀-C₁₈ alkyl dimethyl amine oxides and C₈-C₁₂ alkoxy ethyl dihydroxy ethyl amine oxides.

When included therein, the laundry detergent compositions of the present invention typically comprise from 0.2% to about 15%, preferably from about 1% to about 10% by weight of such semi-polar nonionic surfactants.

### Optional detergent ingredients :

### Dispersant

It has been found that combinations of specific amylase enzyme according to the present invention with a dispersant enhance the overall cleaning and stain removal performance.

It has been found that the use of low molecular weight dispersant in the presence of an α-amylase gives a significant improvement in starch cleaning. Increasing enzyme concentration or dispersant alone do not give improved starch cleaning at lower temperature. However the addition of a dispersant to the α-amylase boosts significantly the starch removal at low temperature, enhancing the removal of the hydrolysed fragments of the starch molecules.

Suitable water-soluble organic salts are the homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 1,000 to 100,000.

Especially, copolymer of acrylate and methylacrylate such as the 480N having a molecular weight of 4000, at a level from 0.5-20% by weight of composition can be added in the detergent compositions of the present invention.

### Other detergent enzymes

The detergent compositions can in addition to specific amylase enzymes and protease further comprise one or more enzymes which provide cleaning performance and/or fabric care benefits.
Said enzymes include enzymes selected from cellulases, hemicellulases, peroxidases, gluco-amylases, other amylases, xylanases, lipases, esterases, cutinases, pectinases, reductases, oxidases, phenoloxidases, lipoxygenases, ligninases, pullulanases, tannases, pentosanases, malanases, β-glucanases, arabinosidases chondroitinase, laccase or mixtures thereof.

A preferred combination is a cleaning composition having a cocktail of conventional applicable enzymes including protease along with amylase, lipase, cutinase and/or cellulase in conjunction with one or more plant cell wall degrading enzymes.

The cellulases usable in the present invention include both bacterial or fungal cellulase. Preferably, they will have a pH optimum of between 5 and 9.5. Suitable cellulases are disclosed in U.S. Patent 4,435,307, Barbesgoard et al, which discloses fungal cellulase produced from Humicola insolens. Suitable cellulases are also disclosed in GB-A-2.075.028; GB-A-2.095.275 and DE-OS-2.247.832.

Examples of such cellulases are cellulases produced by a strain of Humicola insolens (Humicola grisea var. thermoidea), particularly the Humicola strain DSM 1800.
Other suitable cellulases are cellulases originated from Humicola insolens having a molecular weight of about 50KDa, an isoelectric point of 5.5 and containing 415 amino acids. Especially suitable cellulases are the cellulases having color care benefits. Examples of such cellulases are cellulases described in European patent application No. 91202879.2, filed November 6, 1991 (Novo).

Peroxidase enzymes are used in combination with oxygen sources, e.g. percarbonate, perborate, persulfate, hydrogen peroxide, etc. They are used for "solution bleaching", i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro- and bromo-peroxidase.

Peroxidase-containing detergent compositions are disclosed, for example, in PCT International Application WO 89/099813 and in European Patent application EP No. 91202882.6, filed on November 6, 1991.

Said cellulases and/or peroxidases are normally incorporated in the detergent composition at levels from 0.0001% to 2% of active enzyme by weight of the detergent composition.

It has been found that combinations of the specific amylase enzyme with protease, enhance the overall cleaning and stain removal performance.

Suitable proteases are the subtilisins which are obtained from particular strains of *B*. *subtilis* and *B*. *licheniformis* (subtilisin BPN and BPN'). One suitable protease is obtained from a strain of *Bacillus*, having maximum activity throughout the pH range of 8-12, developed and sold as ESPERASE® by Novo Industries A/S of Denmark, hereinafter "Novo". The preparation of this enzyme and analogous enzymes is described in GB 1,243,784 to Novo. Other suitable proteases include ALCALASE®, DURAZYM® and SAVINASE® from Novo and MAXATASE®, MAXACAL®, PROPERASE® and MAXAPEM® (protein engineered Maxacal) from Gist-Brocades. Proteolytic enzymes also encompass modified bacterial serine proteases, such as those described in European Patent Application Serial Number 87 303761.8, filed April 28, 1987 (particularly pages 17, 24 and 98), and which is called herein "Protease B", and in European Patent Application 199,404, Venegas, published October 29, 1986, which refers to a modified bacterial serine protealytic enzyme which is called "Protease A" herein. Suitable is what is called herein "Protease C", which is a variant of an alkaline serine protease from *Bacillus* in which lysine replaced arginine at position 27, tyrosine replaced valine at position 104, serine replaced asparagine at position 123, and alanine replaced threonine at position 274. Protease C is described in EP 90915958:4, corresponding to WO 91/06637, Published May 16, 1991. Genetically modified variants, particularly of Protease C, are also included herein.
A preferred protease referred to as "Protease D" is a carbonyl hydrolase variant having an amino acid sequence not found in nature, which is derived from a precursor carbonyl hydrolase by substituting a different amino acid for a plurality of amino acid residues at a position in said carbonyl hydrolase equivalent to position +76, preferably also in combination with one or more amino acid residue positions equivalent to those selected from the group consisting of +99, +101, +103, +104, +107, +123, +27, +105, +109, +126, +128, +135, +156, +166, +195, +197, +204, +206, +210, +216, +217, +218, +222, +260, +265, and/or +274 according to the numbering of *Bacillus amyloliquefaciens* subtilisin, as described in WO95/10591 and in the patent application of C. Ghosh, et al, "Bleaching Compositions Comprising Protease Enzymes" EP 723 580, filed October 13, 1994.
Also suitable for the present invention are proteases described in patent applications EP 251 446 and WO 91/06637, protease BLAP® described in WO91/02792 and their variants described in WO 95/23221.
See also a high pH protease from Bacillus sp. NCIMB 40338 described in WO 93/18140 A to Novo. Enzymatic detergents comprising protease, one or more other enzymes, and a reversible protease inhibitor are described in WO 92/03529 A to Novo. When desired, a protease having decreased adsorption and increased hydrolysis is available as described in WO 95/07791 to Procter & Gamble. A recombinant trypsin-like protease for detergents suitable herein is described in WO 94/25583 to Novo. Other suitable proteases are described in EP 516 200 by Unilever.
The proteolytic enzymes are incorporated in the detergent compositions of the present invention a level of from 0.0001% to 2%, preferably from 0.001% to 0.2%, more preferably from 0.005% to 0.1% pure enzyme by weight of the composition.

Other preferred enzymes that can be included in the detergent compositions of the present invention include lipases. Suitable lipase enzymes for detergent usage include those produced by microorganisms of the Pseudomonas group, such as Pseudomonas stutzeri ATCC 19.154, as disclosed in British Patent 1,372,034. Suitable lipases include those which show a positive immunological cross-reaction with the antibody of the lipase, produced by the microorganism *Pseudomonas fluorescent* IAM 1057. This lipase is available from Amano Pharmaceutical Co. Ltd., Nagoya, Japan, under the trade name Lipase P "Amano," hereinafter referred to as "Amano-P". Other suitable commercial lipases include Amano-CES, lipases ex *Chromobacter viscosum*, e.g. *Chromobacter viscosum var*. *lipolyticum* NRRLB 3673 from Toyo Jozo Co., Tagata, Japan; Chromobacter viscosum lipases from U.S. Biochemical Corp., U.S.A. and Disoynth Co., The Netherlands, and lipases ex *Pseudomonas gladioli.* Especially suitable lipases are lipases such as M1 Lipase^{R} and Lipomax^{R} (Gist-Brocades) and Lipolase^{R} and Lipolase Ultra^{R}(Novo) which have found to be very effective when used in combination with the compositions of the present invention. Also suitables are the lipolytic enzymes described in EP 258 068, WO 92/05249 and WO 95/22615 by Novo Nordisk and in WO 94/03578, WO 95/35381 and WO 96/00292 by Unilever.
Also suitable are cutinases [EC 3.1.1.50] which can be considered as a special kind of lipase, namely lipases which do not require interfacial activation. Addition of cutinases to detergent compositions have been described in e.g. WO-A-88/09367 (Genencor); WO 90/09446 (Plant Genetic System) and WO 94/14963 and WO 94/14964 (Unilever).
The lipases and/or cutinases are normally incorporated in the detergent composition at levels from 0.0001% to 2% of active enzyme by weight of the detergent composition.

It has been found that combination of specific amylase enzyme according to the present invention with a complementary amylase, enhances the overall cleaning and stain removal performance of the detergent composition of the present invention. In particular, it has been found that the inclusion of a multiple amylase system comprising amylases with different temperature optima significantly improves the cleaning performance over a broad range of temperature, especially from 40°C to 65°C. Indeed, the specific amylases according to the present invention demonstrate improved cleaning properties at low temperature versus complementary amylases such as Termamyl. Combinations of low temperature active with high temperature active amylases is therefore contemplated.

By "complementary" it is meant the addition of one or more amylase suitable for detergency purposes. Examples of complementary amylases (α and/or β) are described below. WO94/02597 and WO95/10603, Novo Nordisk A/S describe cleaning compositions which incorporate mutant amylases. Other amylases known for use in cleaning compositions include both α- and β-amylases. α-Amylases are known in the art and include those disclosed in US Pat. no. 5,003,257; EP 252,666; WO/91/00353; FR 2,676,456; EP 285,123; EP 525,610; EP 368,341; and British Patent specification no. 1,296,839 (Novo). Other suitable amylases are stability-enhanced amylases described in WO94/18314, and WO96/05295, Genencor and amylase variants having additional modification in the immediate parent available from Novo Nordisk A/S, disclosed in WO 95/10603. Also suitable are amylases described in EP 277 216 (Novo Nordisk).
Examples of commercial α-amylases products are Purafect Ox Am® from Genencor and Termamyl®, Ban® , Fungamyl® and Duramyl®, all available from Novo Nordisk A/S Denmark. WO95/26397 describes other suitable amylases : α-amylases characterised by having a specific activity at least 25% higher than the specific activity of Termamyl® at a temperature range of 25°C to 55°C and at a pH value in the range of 8 to 10, measured by the Phadebas® α-amylase activity assay. Suitable are variants of the above enzymes, described in WO96/23873 (Novo Nordisk). Other amylolytic enzymes with improved properties with respect to the activity level and the combination of thermostability and a higher activity level are described in WO95/35382.
Preferred complementary amylases for the present invention are the amylases sold under the tradename Purafect Ox Am^{R} described in WO 94/18314, WO96/05295 sold by Genencor; Termamyl®, Fungamyl®, Ban® and Duramyl®, all available from Novo Nordisk A/S and Maxamyl® by Gist-Brocades.

Said complementary amylase is generally incorporated in the detergent compositions of the present invention a level of from 0.0001% to 2%, preferably from 0.00018% to 0.06%, more preferably from 0.00024% to 0.048% pure enzyme by weight of the composition. Preferably a weight of pure enzyme ratio of specific amylase to the complementary amylase is comprised between 9:1 to 1:9, more preferably between 4:1 to 1:4, and most preferably between 2:1 ans 1:2.

The above-mentioned enzymes may be of any suitable origin, such as vegetable, animal, bacterial, fungal and yeast origin.

Said enzymes are normally incorporated in the detergent composition at levels from 0.0001% to 2% of active enzyme by weight of the detergent composition.

It has been found that combination of specific amylase enzyme according to the present invention with a clorine scavenger, enhance the overall cleaning and stain removal performance. Suitable detergent ingredients that can be added are the enzyme oxidation scavengers which are described in the copending European patent application 92870018.6 filed on January 31, 1992. Examples of such enzyme oxidation scavengers are ethoxylated tetraethylene polyamines.

### Color care benefits

Especially preferred detergent ingredients are combinations with technologies which also provide a type of color care benefit. Examples of these technologies are metallo catalysts for color maintenance. Such metallo catalysts are described in the European patent EP 0 596 184 and in the copending European Patent Application No. 94870206.3.

### Bleaching agent

It has been found that combinations of specific amylase enzyme according to the present invention with a bleach system enhance the overall cleaning and stain removal performances.

Bleach systems that can be included in the detergent compositions of the present invention include bleaching agents such as PB1, PB4 and percarbonate with a particle size of 400-800 microns. These bleaching agent components can include one or more oxygen bleaching agents and, depending upon the bleaching agent chosen, one or more bleach activators. When present oxygen bleaching compounds will typically be present at levels of from about 1% to about 25%. In general, bleaching compounds are optional components in non-liquid formulations, e.g. granular detergents.

The bleaching agent component for use herein can be any of the bleaching agents useful for detergent compositions including oxygen bleaches as well as others known in the art.
The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybytyric acid and diperoxydodecanedioic acid. Such bleaching agents are disclosed in U.S. Patent 4,483,781, U.S. Patent Application 740,446, European Patent Application 0,133,354 and U.S. Patent 4,412,934. Highly preferred bleaching agents also include 6-nonylamino-6-oxoperoxycaproic acid as described in U.S. Patent 4,634,551. Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

The hydrogen peroxide releasing agents can be used in combination with bleach activators such as tetraacetylethylenediamine (TAED), nonanoyloxybenzene-sulfonate (NOBS, described in US 4,412,934), 3,5,-trimethylhexanoloxybenzenesulfonate (ISONOBS, described in EP 120,591) or pentaacetylglucose (PAG), which are perhydrolyzed to form a peracid as the active bleaching species, leading to improved bleaching effect. Also suitable activators are acylated citrate esters.

Useful bleaching agents, including peroxyacids and bleaching systems comprising bleach activators and peroxygen bleaching compounds for use in detergent compositions according to the invention are described in our co-pending application WO 97/00937, WO95/27772, WO95/27773, WO95/27774 and WO95/27775.

The hydrogen peroxide may also be present by adding an enzymatic system (i.e. an enzyme and a substrate therefore) which is capable of generating hydrogen peroxide at the beginning or during the washing and/or rinsing process. Such enzymatic systems are disclosed in EP Patent Application EP 537 381 A filed October 9, 1991.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, detergent compositions will contain about 0.025% to about 1.25%, by weight, of sulfonated zinc phthalocyanine.

### Builder system

The compositions according to the present invention may further comprise a builder system. It has been found that the combination of specific amylase enzyme according to the present invention with builder component, enhance the overall cleaning and stain removal performance.

Any conventional builder system is suitable for use herein including aluminosilicate materials, silicates, polycarboxylates and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.
Another suitable inorganic builder material is layered silicate, e.g. SKS-6 (Hoechst). SKS-6 is a crystalline layered silicate consisting of sodium silicate (Na₂Si₂O₅).
Suitable polycarboxylates containing one carboxy group include lactic acid, glycolic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlegenschrift 2,446,686, and 2,446,687 and U.S. Patent No. 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis,cis-tetracarboxylates, cyclopentadienide pentacarboxylates, 2,3,4,5-tetrahydrofuran - cis, cis, cis-tetracarboxylates, 2,5-tetrahydrofuran -cis - dicarboxylates, 2,2,5,5-tetrahydrofuran - tetracarboxylates, 1,2,3,4,5,6-hexane -hexacar-boxylates and and carboxymethyl derivatives of polyhydric alcohols such as sorbitol, mannitol and xylitol. Aromatic poly-carboxylates include mellitic acid, pyromellitic acid and the phthalic acid derivatives disclosed in British Patent No. 1,425,343.
Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent compositions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt thereof. Examples of such preferred sodium salts of EDDS include Na₂EDDS and Na₄EDDS. Examples of such preferred magnesium salts of EDDS include MgEDDS and Mg₂EDDS. The magnesium salts are the most preferred for inclusion in compositions in accordance with the invention.

Preferred builder systems include a mixture of a water-insoluble aluminosilicate builder such as zeolite A, and a watersoluble carboxylate chelating agent such as citric acid.
Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 10% to 80% by weight of the composition preferably from 20% to 70% and most usually from 30% to 60% by weight.

### Suds suppressor

Another optional ingredient is a suds suppressor, exemplified by silicones, and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the suds suppressor is advantageously releasably incorporated in a water-soluble or water-dispersible, substantially non-surface-active detergent impermeable carrier. Alternatively the suds suppressor can be dissolved or dispersed in a liquid carrier and applied by spraying on to one or more of the other components.
A preferred silicone suds controlling agent is disclosed in Bartollota et al. U.S. Patent 3 933 672. Other particularly useful suds suppressors are the self-emulsifying silicone suds suppressors, described in German Patent Application DTOS 2 646 126 published April 28, 1977. An example of such a compound is DC-544, commercially available from Dow Corning, which is a siloxane-glycol copolymer. Especially preferred suds controlling agent are the suds suppressor system comprising a mixture of silicone oils and 2-alkyl-alcanols. Suitable 2-alkyl-alkanols are 2-bytyl-octanol which are commercially available under the trade name Isofol 12 R.
Such suds suppressor system are described in Copending European Patent application N 92870174.7 filed 10 November, 1992.
Especially preferred silicone suds controlling agents are described in Copending European Patent application N°92201649.8. Said compositions can comprise a silicone/silica mixture in combination with fumed nonporous silica such as Aerosil^{R}.

The suds suppressors described above are normally employed at levels of from 0.001% to 2% by weight of the composition, preferably from 0.01% to 1% by weight.

### Others

Other components used in detergent compositions may be employed, such as soil-suspending agents, soil-release agents, optical brighteners, abrasives, bactericides, tarnish inhibitors, coloring agents, and/or encapsulated or non-encapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid-esters of substituted dicarboxylic acids such as described in US 3,455,838. These acid-ester dextrins are,preferably, prepared from such starches as waxy maize, waxy sorghum, sago, tapioca and potato. Suitable examples of said encapsulating materials include N-Lok manufactured by National Starch. The N-Lok encapsulating material consists of a modified maize starch and glucose. The starch is modified by adding monofunctional substituted groups such as octenyl succinic acid anhydride.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably from 0.75% to 8%, most preferably from 1% to 6% by weight of the composition.

Preferred optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulphonate, monosodium 4',4'' -bis-(2,4-dianilino-s-triazin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6- ylami-no)stilbene-2,2'disulphonate, sodium 2(stilbyl-4''-(naphtho-1',2':4,5)-1,2,3 - triazole-2''-sulphonate and 4,4'-bis(2-sulphostyryl)biphenyl. Highly preferred brighteners are the specific brighteners of copending European Patent application No. EP 753 567 A.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

Soil release agents useful in compositions of the present invention are conventionally copolymers or terpolymers of terephthalic acid with ethylene glycol and/or propylene glycol units in various arrangements. Examples of such polymers are disclosed in the commonly assigned US Patent Nos. 4116885 and 4711730 and European Published Patent Application No. 0 272 033. A particular preferred polymer in accordance with EP-A-0 272 033 has the formula

(CH₃(PEG)₄₃)_{0.75}(POH)_{0.25}[T-PO)_{2.8}(T-PEG)_{0.4}]T(PO-H)_{0.25}((PEG)₄₃CH₃)_{0.75}

where PEG is -(OC₂H₄)O-, PO is (OC₃H₆O) and T is (pcOC₆H₄CO).

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or propane 1-2 diol, thereof consist "secondarily" of such species.

The selected polyesters herein contain about 46% by weight of dimethyl terephthalic acid, about 16% by weight of propane -1.2 diol, about 10% by weight ethylene glycol about 13% by weight of dimethyl sulfobenzoic acid and about 15% by weight of sulfoisophthalic acid, and have a molecular weight of about 3.000. The polyesters and their method of preparation are described in detail in EPA 311 342.

Is is well known in the art that free chlorine in tap water rapidly deactivates the enzymes comprised in detergent compositions. Therefore, using chlorine scavenger such as perborate, ammonium sulfate, sodium sulphite or polyethyleneimine at a level above 0.1% by weight of total composition, in the formulas will provide improved through the wash stability of the specific amylase enzymes. Compositions comprising chlorine scavenger are described in the European patent application 92870018.6 filed January 31, 1992.

### Softening agents

Fabric softening agents can also be incorporated into laundry detergent compositions in accordance with the present invention. These agents may be inorganic or organic in type. Inorganic softening agents are exemplified by the smectite clays disclosed in GB-A-1 400 898 and in USP 5,019,292. Organic fabric softening agents include the water insoluble tertiary amines as disclosed in GB-A1 514 276 and EP-B0 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146.

Levels of smectite clay are normally in the range from 2% to 20%, more preferably from 5% to 15% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

### Dye transfer inhibition

The detergent composition of the present invention can also include compounds for inhibiting dye transfer from one fabric to another of solubilized and suspended dyes encountered during fabric laundering operations involving colored fabrics.

### Polymeric dye transfer inhibiting agents

The detergent compositions according to the present invention also comprise from 0.001% to 10 %, preferably from 0.01% to 2%, more preferably from 0.05% to 1% by weight of polymeric dye transfer inhibiting agents. Said polymeric dye transfer inhibiting agents are normally incorporated into detergent compositions in order to inhibit the transfer of dyes from colored fabrics onto fabrics washed therewith. These polymers have the ability to complex or adsorb the fugitive dyes washed out of dyed fabrics before the dyes have the opportunity to become attached to other articles in the wash.
Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof.
Addition of such polymers also enhances the performance of the enzymes according the invention.

### a) Polyamine N-oxide polymers

The polyamine N-oxide polymers suitable for use contain units having the following structure formula : 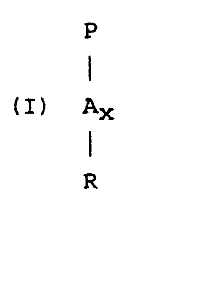 wherein P is a polymerisable unit, whereto the R-N-O group can be attached to or wherein the R-N-O group forms part of the polymerisable unit or a combination of both.
A is NC, 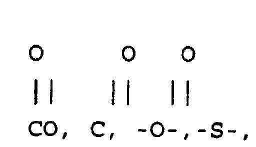 - N- ; x is O or 1;
R are aliphatic, ethoxylated aliphatics, aromatic, heterocyclic or alicyclic groups or any combination thereof whereto the nitrogen of the N-O group can be attached or wherein the nitrogen of the N-O group is part of these groups.

The N-O group can be represented by the following general structures : 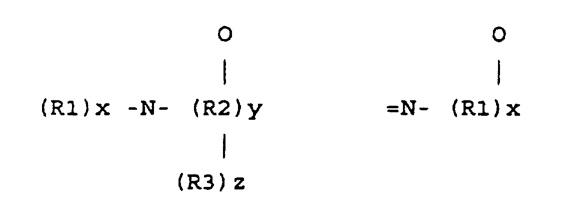 wherein R1, R2, and R3 are aliphatic groups, aromatic, heterocyclic or alicyclic groups or combinations thereof, x or/and y or/and z is 0 or 1 and wherein the nitrogen of the N-O group can be attached or wherein the nitrogen of the N-O group forms part of these groups.

The N-O group can be part of the polymerisable unit (P) or can be attached to the polymeric backbone or a combination of both.
Suitable polyamine N-oxides wherein the N-O group forms part of the polymerisable unit comprise polyamine N-oxides
wherein R is selected from aliphatic, aromatic, alicyclic or heterocyclic groups.
One class of said polyamine N-oxides comprises the group of polyamine N-oxides wherein the nitrogen of the N-O group forms part of the R-group. Preferred polyamine N-oxides are those wherein R is a heterocyclic group such as pyrridine, pyrrole, imidazole, pyrrolidine, piperidine, quinoline, acridine and derivatives thereof.
Another class of said polyamine N-oxides comprises the group of polyamine N-oxides wherein the nitrogen of the N-O group is attached to the R-group.

Other suitable polyamine N-oxides are the polyamine oxides whereto the N-O group is attached to the polymerisable unit.
Preferred class of these polyamine N-oxides are the polyamine N-oxides having the general formula (I) wherein R is an aromatic, heterocyclic or alicyclic groups wherein the nitrogen of the N-O functional group is part of said R group.
Examples of these classes are polyamine oxides wherein R is a heterocyclic compound such as pyrridine, pyrrole, imidazole and derivatives thereof.
Another preferred class of polyamine N-oxides are the polyamine oxides having the general formula (I) wherein R are aromatic, heterocyclic or alicyclic groups wherein the nitrogen of the N-O functional group is attached to said R groups.
Examples of these classes are polyamine oxides wherein R groups can be aromatic such as phenyl.

Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof.

The amine N-oxide polymers of the present invention typically have a ratio of amine to the amine N-oxide of 10:1 to 1:1000000. However the amount of amine oxide groups present in the polyamine oxide polymer can be varied by appropriate copolymerization or by appropriate degree of N-oxidation. Preferably, the ratio of amine to amine N-oxide is from 2:3 to 1:1000000. More preferably from 1:4 to 1:1000000, most preferably from 1:7 to 1:1000000. The polymers of the present invention actually encompass random or block copolymers where one monomer type is an amine N-oxide and the other monomer type is either an amine N-oxide or not. The amine oxide unit of the polyamine N-oxides has a PKa < 10, preferably PKa < 7, more preferred PKa < 6.
The polyamine oxides can be obtained in almost any degree of polymerisation. The degree of polymerisation is not critical provided the material has the desired water-solubility and dye-suspending power.
Typically, the average molecular weight is within the range of 500 to 1000,000; preferably from 1,000 to 50,000, more preferably from 2,000 to 30,000, most preferably from 3,000 to 20,000.

### b) Copolymers of N-vinylpyrrolidone and N-vinylimidazole

The N-vinylimidazole N-vinylpyrrolidone polymers used in the present invention have an average molecular weight range from 5,000-1,000,000, preferably from 5,000-200,000.
Highly preferred polymers for use in detergent compositions according to the present invention comprise a polymer selected from N-vinylimidazole N-vinylpyrrolidone copolymers wherein said polymer has an average molecular weight range from 5,000 to 50,000 more preferably from 8,000 to 30,000, most preferably from 10,000 to 20,000.
The average molecular weight range was determined by light scattering as described in Earth H.G. and Mays J.W. Chemical Analysis Vol 113,"Modern Methods of Polymer Characterization".
Highly preferred N-vinylimidazole N-vinylpyrrolidone copolymers have an average molecular weight range from 5,000 to 50,000; more preferably from 8,000 to 30,000; most preferably from 10,000 to 20,000.

The N-vinylimidazole N-vinylpyrrolidone copolymers characterized by having said average molecular weight range provide excellent dye transfer inhibiting properties while not adversely affecting the cleaning performance of detergent compositions formulated therewith.
The N-vinylimidazole N-vinylpyrrolidone copolymer of the present invention has a molar ratio of N-vinylimidazole to N-vinylpyrrolidone from 1 to 0.2, more preferably from 0.8 to 0.3, most preferably from 0.6 to 0.4 .

### c) Polyvinylpyrrolidone

The detergent compositions of the present invention may also utilize polyvinylpyrrolidone ("PVP") having an average molecular weight of from about 2,500 to about 400,000, preferably from about 5,000 to about 200,000, more preferably from about 5,000 to about 50,000, and most preferably from about 5,000 to about 15,000. Suitable polyvinylpyrrolidones are commercially vailable from ISP Corporation, New York, NY and Montreal, Canada under the product names PVP K-15 (viscosity molecular weight of 10,000), PVP K-30 (average molecular weight of 40,000), PVP K-60 (average molecular weight of 160,000), and PVP K-90 (average molecular weight of 360,000). Other suitable polyvinylpyrrolidones which are commercially available from BASF Cooperation include Sokalan HP 165 and Sokalan HP 12; polyvinylpyrrolidones known to persons skilled in the detergent field (see for example EP-A-262,897 and EP-A-256,696).

### d) Polyvinyloxazolidone :

The detergent compositions of the present invention may also utilize polyvinyloxazolidone as a polymeric dye transfer inhibiting agent. Said polyvinyloxazolidones have an average molecular weight of from about 2,500 to about 400,000, preferably from about 5,000 to about 200,000, more preferably from about 5,000 to about 50,000, and most preferably from about 5,000 to about 15,000.

### e) Polyvinylimidazole :

The detergent compositions of the present invention may also utilize polyvinylimidazole as polymeric dye transfer inhibiting agent. Said polyvinylimidazoles have an average about 2,500 to about 400,000, preferably from about 5,000 to about 200,000, more preferably from about 5,000 to about 50,000, and most preferably from about 5,000 to about 15,000.

### f) Cross-linked polymers :

Cross-linked polymers are polymers whose backbone are interconnected to a certain degree; these links can be of chemical or physical nature, possibly with active groups n the backbone or on branches; cross-linked polymers have been described in the Journal of Polymer Science, volume 22, pages 1035-1039.

In one embodiment, the cross-linked polymers are made in such a way that they form a three-dimensional rigid structure, which can entrap dyes in the pores formed by the three-dimensional structure. In another embodiment, the cross-linked polymers entrap the dyes by swelling.

Such cross-linked polymers are described in the copending patent application 94870213.9

### Method of washing

The cleaning compositions according to the invention can be liquid, paste, gels, bars, tablets, powder or granular forms. Granular compositions can also be in "compact " form, the liquid compositions can also be in a "concentrated" form.

The compositions of the invention may be formulated as hand and machine laundry detergent compositions including laundry additive compositions and compositions suitable for use in the pretreatment or soaking of stained fabric, rinse added fabric softener compositions.

The compositions of the invention may be used in essentially any washing or cleaning methods, including soaking methods, pretreatment methods and methods with rinsing steps for which a separate rinse aid composition may be added.

The process described herein comprises contacting fabrics with a laundering solution in the usual manner and exemplified hereunder.

The process of the invention is conveniently carried out in the course of the cleaning process. The method of cleaning is preferably carried out at 5 °C to 95 °C, especially between 10°C and 60°C. However, specific amylase enzymes within the specified enzymatic concentration, have demonstrated superior starch cleaning even for wash cycles occuring at very low temperatures (between 10°C and 25°C). The pH of the treatment solution is preferably from 7 to 11.

A preferred machine dishwashing method comprises treating soiled articles with an aqueous solution of the machine diswashing or rinsing composition. A conventional effective amount of the machine dishwashing composition means from 8-60 g of product dissolved or dispersed in a wash volume from 3-10 litres.
According to a manual dishwashing method, soiled dishes are contacted with an effective amount of the diswashing composition, typically from 0.5-20g (per 25 dishes being treated). Preferred manual dishwashing methods include the application of a concentrated solution to the surfaces of the dishes or the soaking in large volume of dilute solution of the detergent composition.

The compositions of the invention may also be formulated as hard surface cleaner compositions.

The following examples are meant to exemplify compositions of the present invention, byt are not necessarily meant to limit or otherwise define the scope of the invention.

In the detergent compositions, the abbreviated component identifications have the following meanings:
- LAS :: Sodium linear C₁₂ alkyl benzene sulphonate
- TAS :: Sodium tallow alkyl sulphate
- XYAS :: Sodium C_{1X} - C_{1Y} alkyl sulfate
- SAS :: C₁₂-C₁₄ secondary (2,3) alkyl sulfate in the form of the sodium salt.
- APG :: Alkyl polyglycoside surfactant of formula C₁₂ - (glycosyl)ₓ, where x is 1.5,
- AEC :: Alkyl ethoxycarboxylate surfactant of formula C₁₂ ethoxy (2) carboxylate.
- SS :: Secondary soap surfactant of formula 2-bytyl octanoic acid
- 25EY :: A C₁₂-C₁₅ predominantly linear primary alcohol condensed with an average of Y moles of ethylene oxide
- 45EY :: A C₁₄ - C₁₅ predominantly linear primary alcohol condensed with an average of Y moles of ethylene oxide
- XYEZS :: C_{1X} - C_{1Y} sodium alkyl sulfate condensed with an average of Z moles of ethylene oxide per mole
- Nonionic :: C₁₃-C₁₅ mixed ethoxylated/propoxylated fatty alcohol with an average degree of ethoxylation of 3.8 and an average degree of propoxylation of 4.5 sold under the tradename Plurafax LF404 by BASF Gmbh
- CFAA :: C₁₂-C₁₄ alkyl N-methyl glucamide
- TFAA :: C₁₆-C₁₈ alkyl N-methyl glucamide.
- Silicate :: Amorphous Sodium Silicate (SiO₂:Na₂O ratio = 2.0)
- NaSKS-6 :: Crystalline layered silicate of formula δ-Na₂Si₂O₅
- Carbonate :: Anhydrous sodium carbonate
- Metasilicate :: Sodium metasilicate (SiO₂:Na₂O ratio = 2.0)
- Phosphate or STPP :: Sodium tripolyphosphate
- MA/AA :: Copolymer of 1:4 maleic/acrylic acid, average molecular weight about 80,000
- PA30 :: Polyacrylic acid of average molecular weight of approximately 8,000.
- Terpolymer :: Terpolymer of average molecular weight approx. 7,000, comprising acrylic:maleic:ethylacrylic acid monomer units at a weight ratio of 60:20:20
- 480N :: Random copolymer of 3:7 acrylic/methacrylic acid, average molecular weight about 3,500.
- Polyacrylate :: Polyacrylate homopolymer with an average molecular weight of 8,000 sold under the tradename PA30 by BASF GmbH
- Zeolite A :: Hydrated Sodium Aluminosilicate of formula Na₁₂(AlO₂SiO₂)₁₂. 27H₂O having a primary particle size in the range from 1 to 10 micrometers
- Zeolite MAP :: Alkali metal alumino-silicate of the zeolite P type having a silicon to aluminium ratio not greater than 1.33
- Citrate :: Tri-sodium citrate dihydrate
- Citric :: Citric Acid
- Perborate :: Anhydrous sodium perborate monohydrate bleach, empirical formula NaBO₂.H₂O₂
- PB4 :: Anhydrous sodium perborate tetrahydrate.
- Percarbonate :: Anhydrous sodium percarbonate bleach of empirical formula 2Na₂CO₃.3H₂O₂
- TAED :: Tetraacetyl ethylene diamine
- Paraffin :: Paraffin oil sold under the tradename Winog 70 by Wintershall.
- Pectinase :: Pectolytic enzyme sold under the tradename Pectinex AR by Novo Nordisk A/S.
- Xylanase :: Xylanolytic enzyme sold under the tradenames Pulpzyme HB or SP431 by Novo Nordisk A/S or Lyxasan (Gist-Brocades) or Optipulp or Xylanase (Solvay).
- Protease :: Proteolytic enzyme sold under the tradename Savinase, Alcalase, Durazym by Novo Nordisk A/S, Maxacal, Maxapem sold by Gist-Brocades and proteases described in patents WO91/06637 and/or WO95/10591 and/or EP 251 446.
- Amylase :: Specific amylase enzyme according to the present invention.
- Complementary : amylase: Amylolytic enzyme sold under the tradename Purafect Ox Am^{R} described in WO 94/18314, WO96/05295 sold by Genencor; Termamyl®, Fungamyl®, Ban® and Duramyl®, all available from Novo Nordisk A/S and Maxamyl® by Gist-Brocades.
- Lipase :: Lipolytic enzyme sold under the tradename Lipolase, Lipolase Ultra by Novo Nordisk A/S
- Peroxidase :: Peroxidase enzyme
- Cellulase :: Cellulosic enzyme sold under the tradename Carezyme or Celluzyme by Novo Nordisk A/S.
- CMC :: Sodium carboxymethyl cellulose
- HEDP :: 1,1-hydroxyethane diphosphonic acid
- DETPMP :: Diethylene triamine penta (methylene phosphonic acid), marketed by Monsanto under the Trade name Dequest 2060.
- PAAC :: pentaamine acetate cobalt (III) sal.
- BzP :: Benzoyl peroxide.
- PVP :: Polyvinyl pyrrolidone polymer.
- PVNO :: Poly(4-vinylpyridine)-N-Oxide.
- Soil Release :: Sulfonated poly-ethoxy/propoxy end capped ester oligomer.
- Polymer EDDS :: Ethylenediamine -N, N'- disuccinic acid, [S, S] isomer in the form of the sodium salt.
- Suds Suppressor :: 25% paraffin wax Mpt 50°C, 17% hydrophobic silica, 58% paraffin oil.
- Granular Suds : Suppressor: 12% Silicone/silica, 18% stearyl alcohol,70% starch in granular form
- SCS :: Sodium cumene sulphonate
- Sulphate :: Anhydrous sodium sulphate.
- HMWPEO :: High molecular weight polyethylene oxide
- PGMS :: Polyglycerol monostearate having a tradename of Radiasurf 248
- TAE 25 :: Tallow alcohol ethoxylate (25)
- PEG(-6) :: Polyethylene glycol (having a molecular weight of 600).
- BTA :: Benzotriazole
- Bismuth nitrate :: Bismuth nitrate salt
- NaDCC :: Sodium dichloroisocyanurate
- KOH :: 100% Active solution of Potassium Hydroxide
- PZ-Base :: Sugar matrix protected zeolite - perfume carrier.
- pH :: Measured as a 1% solution in distilled water at 20°C.

### Example 1

Granular fabric cleaning compositions in accord with the invention were prepared as follows:

| | **I** | **II** | **III** | **IV** | **V** |
|---|---|---|---|---|---|
| LAS | 18.0 | 9.4 | 21.0 | 21.5 | 21.5 |
| AS | - | 11.3 | - | 6.55 | 6.55 |
| AES | - | 1.00 | - | - | - |
| XYEZS | 1.5 | - | - | - | - |
| Alkyl C₁₂₋₁₄ methyl | 0.7 | - | - | 0.5 | 0.5 |
| dihydroxyethyl | | | | | |
| ammonium chloride | | | | | |
| C45E7 | 0.9 | - | - | - | - |
| NI | - | 1.5 | 1.2 | 3.3 | 3.3 |
| Phosphate | 22.5 | - | 40 | - | - |
| MA/AA | 1.0 | - | - | - | - |
| MA/AA 4/6, MW 11000 | - | - | - | 7.10 | 7.10 |
| Soil release polymer | 0.2 | 0.4 | 0.3 | 0.3 | 0.3 |
| CMC | 0.75 | - | 0.3 | - | - |
| Polyethyleneimine (MW 1800) | 0.25 | - | 1.00 | - | - |
| Na-SKS-6 | - | - | - | 3.3 | 3.3 |
| Aluminosilicate | - | 27.8 | 8.00 | 8.4 | 8.4 |
| Carbonate | 5.0 | 26.4 | 21.7 | 18.9 | 18.9 |
| Silicate | 7.6 | 14.7 | 2.0 | 11.7 | 11.7 |
| sulfate | - | 5.4 | - | 5.2 | 5.2 |
| PVNO | - | - | 0.5 | - | - |
| Polyacrylate | - | 2.3 | - | - | - |
| PEG 4000 | - | 1.6 | - | 0.2 | 0.2 |
| Suds suppressor | - | 0.60 | 0.25 | 0.4 | 0.4 |
| Specific amylase enzymes | 0.003 | 0.01 | 0.008 | 0.0077 | 0.008 |
| Complementary amylase | - | - | - | - | 0.004 |
| Protease | 0.006 | 0.01 | 0.014 | 0.03 | 0.03 |
| Lipase | 0.002 | - | 0.024 | - | - |
| Cellulase | 0.0005 | 0.004 | - | - | - |
| DTPA | - | 0.8 | - | - | - |
| PB1 | - | 1.0 | 0.5 | 4.0 | 4.0 |
| NOBS | - | - | - | 4.0 | 4.0 |
| HEDP | - | - | 0.5 | - | - |
| DETPMP | 0.6 | - | - | - | - |
| MgSO4 | 0.8 | - | - | - | - |
| PZ-Base | - | - | 1.5 | - | - |
| Brightener 49 | 0.05 | - | 0.05 | 0.21 | 0.21 |
| Brightener 24 | - | 0.17 | - | - | - |
| Brightener 15 | 0.15 | - | 0.05 | - | - |
| Brightener 3 | - | - | - | 0.10 | 0.10 |
| Perfume | - | 0.4 | 0.3 | 0.25 | 0.25 |
| Water & minors | Up to 100% | | | | |

The level of the enzymes comprised in such composition are expressed in pure enzyme by weight of total composition.

### Example 2

Granular fabric cleaning compositions I to IV in accord with the invention were prepared as follows. Composdions V and VI are comparative.

| | **I** | **II** | **III** | **IV** | **V** | **VI** |
|---|---|---|---|---|---|---|
| LAS | 22.0 | 22.0 | 22.0 | 22.0 | 22.0 | 22.0 |
| Phosphate | 23.0 | 23.0 | 23.0 | 23.0 | 23.0 | 23.0 |
| Carbonate | 23.0 | 23.0 | 23.0 | 23.0 | 23.0 | 23.0 |
| Silicate | 14.0 | 14.0 | 14.0 | 14.0 | 14.0 | 14.0 |
| Zeolite A | 8.2 | 8.2 | 8.2 | 8.2 | 8.2 | 8.2 |
| DETPMP | 0.4 | 0.4 | 0.4 | 0.4 | 0.4 | 0.4 |
| Sodium sulfate | 5.5 | 5.5 | 5.5 | 5.5 | 5.5 | 5.5 |
| Specific amylase enzyme | 0.005 | 0.02 | 0.01 | 0.01 | 0.02 | 0.02 |
| Complementary amylase | - | - | - | - | - | 0.005 |
| Protease | 0.01 | 0.02 | 0.01 | 0.005 | - | - |
| Pectinase | 0.02 | - | - | - | - | - |
| Xylanase | - | - | 0.01 | 0.02 | - | - |
| Lipase | 0.005 | 0.01 | - | - | - | - |
| Cellulase | 0.001 | - | - | 0.001 | - | - |
| Water & minors | Up to 100% | | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 3

Granular fabric cleaning compositions I to IV in accord with the invention were prepared as follows. Compositions V and VI are comparative.

| | **I** | **II** | **III** | **IV** | **V** | **VI** |
|---|---|---|---|---|---|---|
| LAS | 12.0 | 12.0 | 12.0 | 12.0 | 12.0 | 12.0 |
| Zeolite A | 26.0 | 26.0 | 26.0 | 26.0 | 26.0 | 26.0 |
| SS | 4.0 | 4.0 | 4.0 | 4.0 | 4.0 | 4.0 |
| SAS | 5.0 | 5.0 | 5.0 | 5.0 | 5.0 | 5.0 |
| Citrate | 5.0 | 5.0 | 5.0 | 5.0 | 5.0 | 5.0 |
| Sodium Sulfate | 17.0 | 17.0 | 17.0 | 28.0 | 17.0 | 17.0 |
| Perborate | 16.0 | 16.0 | 16.0 | - | 16.0 | 16.0 |
| TAED | 5.0 | 5.0 | 5.0 | - | 5.0 | 5.0 |
| Protease | 0.06 | 0.03 | 0.02 | 0.08 | - | - |
| Lipase | 0.005 | 0.01 | - | - | - | - |
| Complementary amylase | - | - | - | - | - | 0.01 |
| Specific amylase enzyme | 0.01 | 0.015 | 0.01 | 0.02 | 0.005 | 0.005 |
| Water & minors | Up to 100% | | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 4

Granular fabric cleaning compositions in accord with the invention which are especially useful in the laundering of coloured fabrics were prepared as follows :

| | **I** | **II** | **III** | **IV** |
|---|---|---|---|---|
| LAS | 11.4 | 10.7 | - | - |
| TAS | 1.8 | 2.4 | - | - |
| TFAA | - | - | 4.0 | 4.0 |
| 45AS | 3.0 | 3.1 | 10.0 | 10.0 |
| 45E7 | 4.0 | 4.0 | - | - |
| 25E3S | - | - | 3.0 | 3.0 |
| 68E11 | 1.8 | 1.8 | - | - |
| 25E5 | - | - | 8.0 | 8.0 |
| Citrate | 14.0 | 15.0 | 7.0 | 7.0 |
| Carbonate | - | - | 10 | 10 |
| Citric | 3.0 | 2.5 | 3.0 | 3.0 |
| Zeolite A | 32.5 | 32.1 | 25.0 | 25.0 |
| Na-SKS-6 | - | - | 9.0 | 9.0 |
| MA/AA | 5.0 | 5.0 | 5.0 | 5.0 |
| DETPMP | 1.0 | 0.2 | 0.8 | 0.8 |
| Protease | 0.02 | 0.02 | 0.01 | 0.01 |
| Specific amylase enzyme | 0.03 | 0.03 | 0.005 | 0.005 |
| Complementary amylase | - | - | - | 0.02 |
| Silicate | 2.0 | 2.5 | - | - |
| Sulphate | 3.5 | 5.2 | 3.0 | 3.0 |
| PVP | 0.3 | 0.5 | - | - |
| Poly (4-vinylpyridine)-N-oxide/copolymer of vinylimidazole and vinylpyrrolidone | - | - | 0.2 | 0.2 |
| Perborate | 0.5 | 1.0 | - | - |
| Peroxidase | 0.01 | 0.01 | - | - |
| Phenol sulfonate | 0.1 | 0.2 | - | - |
| Water/Minors | Up to 100% | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 5

Granular fabric cleaning compositions in accord with the invention were prepared as follows:

| | **I** | **II** | **III** |
|---|---|---|---|
| LAS | 6.5 | 8.0 | 8.0 |
| Sulfate | 15.0 | 18.0 | 18.0 |
| Zeolite A | 26.0 | 22.0 | 22.0 |
| Sodium nitrilotriacetate | 5.0 | 5.0 | 5.0 |
| PVP | 0.5 | 0.7 | 0.7 |
| TAED | 3.0 | 3.0 | 3.0 |
| Boric acid | 4.0 | - | - |
| Perborate | 0.5 | 1.0 | 1.0 |
| Phenol sulphonate | 0.1 | 0.2 | 0.2 |
| Protease | 0.06 | 0.02 | 0.02 |
| Silicate | 5.0 | 5.0 | 5.0 |
| Carbonate | 15.0 | 15.0 | 15.0 |
| Peroxidase | 0.1 | 0.1 | 0.1 |
| Pectinase | 0.02 | - | - |
| Cellulase | 0.005 | 0.002 | 0.002 |
| Lipase | 0.01 | - | - |
| Complementary amylase | - | - | 0.01 |
| Specific amylase enzyme | 0.01 | 0.01 | 0.01 |
| Water/minors | Up to 100% | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 6

A compact granular fabric cleaning compositions in accord with the invention were prepared as follows:

| | **I** | **II** |
|---|---|---|
| 45AS | 8.0 | 8.0 |
| 25E3S | 2.0 | 2.0 |
| 25E5 | 3.0 | 3.0 |
| 25E3 | 3.0 | 3.0 |
| TFAA | 2.5 | 2.5 |
| Zeolite A | 17.0 | 17.0 |
| NaSKS-6 | 12.0 | 12.0 |
| Citric acid | 3.0 | 3.0 |
| Carbonate | 7.0 | 7.0 |
| MA/AA | 5.0 | 5.0 |
| CMC | 0.4 | 0.4 |
| Poly (4-vinylpyridine)-N-oxide/ | 0.2 | 0.2 |
| copolymer of vinylimidazole and | | |
| vinylpyrrolidone | | |
| Protease | 0.05 | 0.05 |
| Lipase | 0.005 | 0.005 |
| Cellulase | 0.001 | 0.001 |
| Specific amylase enzyme | 0.01 | 0.01 |
| Complementary amylase | - | 0.005 |
| TAED | 6.0 | 6.0 |
| Percarbonate | 22.0 | 22.0 |
| EDDS | 0.3 | 0.3 |
| Granular suds suppressor | 3.5 | 3.5 |
| water/minors | | Up to 100% |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 7

A granular fabric cleaning compositions in accord with the invention which provide "softening through the wash" capability were prepared as follows:

| | **I** | **II** | **III** |
|---|---|---|---|
| 45AS | - | 10.0 | 10.0 |
| LAS | 7.6 | - | - |
| 68AS | 1.3 | - | - |
| 45E7 | 4.0 | - | - |
| 25E3 | - | 5.0 | 5.0 |
| Coco-alkyl-dimethyl hydroxy-ethyl ammonium chloride | 1.4 | 1.0 | 1.0 |
| Citrate | 5.0 | 3.0 | 3.0 |
| Na-SKS-6 | - | 11.0 | 11.0 |
| Zeolite A | 15.0 | 15.0 | 15.0 |
| MA/AA | 4.0 | 4.0 | 4.0 |
| DETPMP | 0.4 | 0.4 | 0.4 |
| Perborate | 15.0 | - | - |
| Percarbonate | - | 15.0 | 15.0 |
| TAED | 5.0 | 5.0 | 5.0 |
| Smectite clay | 10.0 | 10.0 | 10.0 |
| HMWPEO | - | 0.1 | 0.1 |
| Protease | 0.02 | 0.01 | 0.01 |
| Lipase | 0.02 | 0.01 | 0.01 |
| Specific amylase enzyme | 0.03 | 0.005 | 0.005 |
| Complementary amylase | - | - | 0.02 |
| Cellulase | 0.001 | - | - |
| Silicate | 3.0 | 5.0 | 5.0 |
| Carbonate | 10.0 | 10.0 | 10.0 |
| Granular suds suppressor | 1.0 | 4.0 | 4.0 |
| CMC | 0.2 | 0.1 | 0.1 |
| Water/minors | Up to 100% | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 8

Heavy duty liquid fabric cleaning compositions suitable for use in the pretreatment of stained fabrics, and for use in a machine laundering method, in accord with the invention were prepared as follows:

| | **I** | **II** | **III** | **IV** | **V** | **VI** |
|---|---|---|---|---|---|---|
| 24AS | 20.0 | 20.0 | 20.0 | 20.0 | 20.0 | 20.0 |
| SS | 5.0 | 5.0 | 5.0 | 5.0 | 5.0 | 5.0 |
| Citrate | 1.0 | 1.0 | 1.0 | 1.0 | 1.0 | 1.0 |
| 12E₃ | 13.0 | 13.0 | 13.0 | 13.0 | 13.0 | 13.0 |
| Monethanolamine | 2.5 | 2.5 | 2.5 | 2.5 | 2.5 | 2.5 |
| Protease | 0.005 | 0.03 | 0.02 | 0.04 | 0.01 | 0.01 |
| Lipase | 0.002 | 0.01 | 0.02 | - | 0.004 | 0.004 |
| Specific amylase enz. | 0.005 | 0.005 | 0.001 | 0.01 | 0.004 | 0.004 |
| Complementary amylase | - | - | - | - | - | 0.008 |
| Cellulase | 0.04 | - | 0.01 | - | - | - |
| Pectinase | 0.02 | 0.02 | - | - | - | - |
| Water/propylene glycol/ethanol (100:1:1) | | | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 9

Heavy duty liquid fabric cleaning compositions in accord with the invention were prepared as follows:

| | **I** | **II** | **III** | **IV** | **V** |
|---|---|---|---|---|---|
| LAS acid form | - | - | 25.0 | - | - |
| C₁₂₋₁₄ alkenyl succinic | 3.0 | 8.0 | 10.0 | - | - |
| acid | | | | | |
| Citric acid | 10.0 | 15.0 | 2.0 | 2.0 | 2.0 |
| 25AS acid form | 8.0 | 8.0 | - | 15.0 | 15.0 |
| 25AE2S acid form | - | 3.0 | - | 4.0 | 4.0 |
| 25AE7 | - | 8.0 | - | 6.0 | 6.0 |
| 25AE3 | 8.0 | - | - | - | - |
| CFAA | - | - | - | 6.0 | 6.0 |
| DETPMP | 0.2 | - | 1.0 | 1.0 | 1.0 |
| Fatty acid | - | - | - | 10.0 | 10.0 |
| Oleic acid | 1.8 | - | 1.0 | - | - |
| Ethanol | 4.0 | 4.0 | 6.0 | 2.0 | 2.0 |
| Propanediol | 2.0 | 2.0 | 6.0 | 10.0 | 10.0 |
| Protease | 0.02 | 0.02 | 0.02 | 0.01 | 0.01 |
| Specific amylase enzyme | 0.005 | 0.01 | 0.005 | 0.01 | 0.01 |
| Complementary amylase | - | - | - | - | 0.01 |
| Coco-alkyl dimethyl | - | - | 3.0 | - | - |
| hydroxy ethyl ammonium | | | | | |
| chloride | | | | | |
| Smectite clay | - | - | 5.0 | - | - |
| PVP | 1.0 | 2.0 | - | - | - |
| Perborate | - | 1.0 | - | - | - |
| Phenol sulphonate | - | 0.2 | - | - | - |
| Peroxidase | - | 0.01 | - | - | - |
| NaOH | | | Up to pH 7.5 | | |
| Water / minors | | | Up to 100% | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Comparative Example 10

The following rinse added fabric softener compositions, were prepared (parts by weight).

| | **I** | **II** |
|---|---|---|
| Softener active | 24.5 | 24.5 |
| PGMS | 2.0 | 2.0 |
| TAE 25 | 1.5 | 1.5 |
| Specific amylase enzyme | 0.001 | 0.001 |
| Complementary amylase | - | 0.0007 |
| Cellulase | 0.001 | 0.001 |
| HCL | 0.12 | 0.12 |
| Antifoam agent | 0.019 | 0.019 |
| Blue dye | 80ppm | 80ppm |
| CaCl₂ | 0.35 | 0.35 |
| Perfume | 0.90 | 0.90 |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 11

Syndet bar fabric cleaning compositions I and III to V in accord with the invention were prepared as follows. Composition II is comparative

| | **I** | **II** | **III** | **IV** | **V** |
|---|---|---|---|---|---|
| C12-16 alkyl sulfate, Na | 20.0 | 20.0 | 20.0 | 20.0 | 20.0 |
| C12-14 N-methyl glucamide | 5.0 | 5.0 | 5.0 | 5.0 | 5.0 |
| C11-13 alkyl benzene | 10.0 | 10.0 | 10.0 | 10.0 | 10.0 |
| sulphonate, Na | | | | | |
| Sodium carbonate | 25.0 | 25.0 | 25.0 | 25.0 | 25.0 |
| Sodium pyrophosphate | 7.0 | 7.0 | 7.0 | 7.0 | 7.0 |
| Sodium tripolyphosphate | 7.0 | 7.0 | 7.0 | 7.0 | 7.0 |
| Zeolite A | 5.0 | 5.0 | 5.0 | 5.0 | 5.0 |
| Carboxymethylcellulose | 0.2 | 0.2 | 0.2 | 0.2 | 0.2 |
| Polyacrylate (MW 1400) | 0.2 | 0.2 | 0.2 | 0.2 | 0.2 |
| Coconut monethanolamide | 5.0 | 5.0 | 5.0 | 5.0 | 5.0 |
| Specific amylase enzyme | 0.01 | 0.02 | 0.01 | 0.01 | 0.01 |
| Complementary amylase | - | - | - | - | 0.005 |
| Protease | 0.3 | - | 0.5 | 0.05 | 0.05 |
| Brightener, perfume | 0.2 | 0.2 | 0.2 | 0.2 | 0.2 |
| CaSO4 | 1.0 | 1.0 | 1.0 | 1.0 | 1.0 |
| MgSO4 | 1.0 | 1.0 | 1.0 | 1.0 | 1.0 |
| Water | 4.0 | 4.0 | 4.0 | 4.0 | 4.0 |
| Filler* : balance to 100% | | | | | |

| | | | | | |
|---|---|---|---|---|---|
| *Can be selected from convenient materials such as CaCO3, talc, clay (Kaolinite, Smectite), silicates, and the like. | | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 12

The following compact high density (0.96Kg/l) dishwashing detergent compositions I to VI were prepared in accord with the invention:

| | **I** | **II** | **III** | **IV** | **V** | **VI** | **VII** | **VIII** |
|---|---|---|---|---|---|---|---|---|
| STPP | - | - | 48.8 | 37.4 | - | - | - | - |
| Citrate | 32.9 | 17.0 | - | - | 17.0 | 25.4 | 25.4 | 25.4 |
| Carbonate | - | 17.5 | - | 20.0 | 20.0 | 25.0 | 25.0 | 23.0 |
| Silicate | 33.0 | 14.8 | 20.4 | 14.8 | 14.8 | - | - | - |
| Metasilicate | - | 2.50 | 2.50 | - | - | - | - | 2.50 |
| PB1 | 1.9 | 9.7 | 7.8 | 14.3 | 9.7 | - | - | - |
| PB4 | 8.6 | - | - | - | - | - | - | - |
| Percarbonate | - | - | - | - | - | 6.7 | 6.7 | 6.7 |
| Nonionic | 1.5 | 2.0 | 1.5 | 1.5 | 2.0 | 2.6 | 2.6 | 2.6 |
| TAED | 4.8 | 2.4 | 2.4 | - | - | 4.0 | 4.0 | 4.0 |
| HEDP | 0.8 | 1.0 | 0.46 | - | 0.8 | - | - | - |
| DETPMP | 0.65 | 0.65 | - | - | - | - | - | - |
| PAAC | - | - | - | 0.2 | - | - | - | - |
| BzP | - | - | - | 4.4 | - | - | - | - |
| Paraffin | 0.5 | 0.5 | 0.5 | 0.5 | - | 0.2 | 0.2 | 0.2 |
| Protease | .075 | 0.05 | 0.1 | 0.1 | 0.08 | 0.01 | 0.01 | 0.01 |
| Lipase | - | .001 | - | .005 | - | - | - | - |
| Specific | 0.01 | .005 | .015 | .015 | .005 | .0025 | .005 | 0.01 |
| Amylase Enzyme | | | | | | | | |
| Complementary | - | - | - | - | - | - | .033 | .007 |
| amylase | | | | | | | | |
| BTA | 0.3 | 0.3 | 0.3 | 0.3 | - | - | - | - |
| Bismuth | - | 0.3 | - | - | - | - | - | - |
| Nitrate | | | | | | | | |
| PA30 | 4.0 | - | - | - | - | - | - | - |
| Terpolymer | - | - | - | 4.0 | - | - | - | - |
| 480N | - | 6.0 | 2.8 | - | 6.7 | - | - | - |
| Sulphate | 7.1 | 20.8 | 8.4 | - | 26.2 | 1.0 | 1.0 | 1.0 |
| pH (1% solution) | 10.0 | 11.0 | 10.9 | 10.8 | 10.9 | 9.6 | 9.6 | 9.6 |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 13

The following granular dishwashing detergent compositions examples I to III and V of bulk density 1.02Kg/L were prepared in accord with the invention. Compositions IV and VI to VIII are comparative

| | **I** | **II** | **III** | **IV** | **V** | **VI** | **VII** | **VIII** |
|---|---|---|---|---|---|---|---|---|
| STPP | 30.0 | 30.0 | 30.0 | 27.9 | 34.5 | 26.7 | 26.7 | 26.7 |
| Carbonate | 30.5 | 30.5 | 30.5 | 23.0 | 30.5 | 2.8 | 2.8 | 2.0 |
| Silicate | 7.4 | 7.4 | 7.4 | 12.0 | 8.0 | 20.3 | 20.3 | 18.3 |
| Metasilicate | - | - | - | - | - | - | - | 2.5 |
| PB1 | 4.4 | 4.4 | 4.4 | - | 4.4 | - | - | - |
| NaDCC | - | - | - | 2.0 | - | 1.5 | 1.5 | 1.5 |
| Nonionic | 0.75 | 0.75 | 0.75 | 1.9 | 1.2 | 0.5 | 0.5 | 0.5 |
| TAED | 1.0 | 1.0 | - | - | 1.0 | - | - | - |
| PAAC | - | - | .004 | - | - | - | - | - |
| BzP | - | 1.4 | - | - | - | - | - | - |
| Paraffin | 0.25 | 0.25 | 0.25 | - | - | - | - | - |
| Protease | 0.05 | 0.05 | 0.05 | - | 0.1 | - | - | - |
| Lipase | .005 | - | .001 | - | - | - | - | - |
| Specific Amylase | .003 | .001 | 0.01 | 0.02 | 0.01 | .015 | 0.02 | 0.02 |
| Enzyme | | | | | | | | |
| Complementary | - | - | - | - | - | - | .013 | .008 |
| amylase | | | | | | | | |
| BTA | 0.15 | - | 0.15 | - | - | - | - | - |
| Sulphate | 23.9 | 23.9 | 23.9 | 31.4 | 17.4 | - | - | - |
| pH (1% solution) | 10. | 10.8 | 10.8 | 10.7 | 10.7 | 12.3 | 12.3 | 12.3 |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 14

The following detergent composition tablets of 25g weight were prepared in accord with the present invention by compression of a granular dishwashing detergent composition at a pressure of 13KN/cm² using a standard 12 head rotary press:

| | **I** | **II** | **III** | **IV** | **V** |
|---|---|---|---|---|---|
| STPP | - | 48.8 | 47.5 | 47.5 | 47.5 |
| Citrate | 26.4 | - | - | - | - |
| Carbonate | - | 5.0 | - | - | - |
| Silicate | 26.4 | 14.8 | 25.0 | 25.0 | 22.5 |
| Metasilicate | - | - | - | - | 2.5 |
| Protease | 0.03 | 0.075 | 0.01 | 0.01 | 0.01 |
| Lipase | 0.005 | - | - | - | - |
| Specific Amylase | 0.01 | 0.005 | 0.001 | 0.01 | 0.01 |
| Enzyme | | | | | |
| Complementary amylase | - | - | - | 0.007 | 0.005 |
| PB1 | 1.6 | 7.8 | - | - | - |
| PB4 | 6.9 | - | 11.4 | 11.4 | 11.40 |
| Nonionic | 1.2 | 2.0 | 1.1 | 1.1 | 1.10 |
| TAED | 4.3 | 2.4 | 0.8 | 0.8 | 0.80 |
| HEDP | 0.7 | - | - | - | - |
| DETPMP | 0.6 | - | - | - | - |
| Paraffin | 0.4 | 0.5 | - | - | - |
| BTA | 0.2 | 0.3 | - | - | - |
| PA30 | 3.2 | - | - | - | - |
| Sulphate | 25.0 | 14.7 | 3.2 | 3.2 | 3.2 |
| pH (1% solution) | 10.6 | 10.6 | 11.0 | 11.0 | 11.0 |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 15

The following liquid dishwashing detergent compositions in accord with the present invention I to II, of density 1.40Kg/L were prepared:

| | **I** | **II** | **III** | **IV** |
|---|---|---|---|---|
| STPP | 33.3 | 20.0 | 20.0 | 20.0 |
| Carbonate | 2.7 | 2.0 | 2.0 | 1.0 |
| Silicate | - | 4.4 | 4.4 | 3.0 |
| Metasilicate | - | - | - | 2.5 |
| NaDCC | 1.1 | 1.15 | 1.15 | 1.15 |
| Nonionic | 2.5 | 1.0 | 1.0 | 1.0 |
| Paraffin | 2.2 | - | - | - |
| Protease | 0.03 | 0.02 | 0.02 | 0.02 |
| Specific Amylase Enzyme | 0.005 | 0.0025 | 0.0025 | 0.0038 |
| Complemetary amylase | - | - | 0.0017 | 0.0025 |
| 480N | 0.5 | 4.0 | 4.0 | 4.0 |
| KOH | - | 6.0 | 6.0 | 6.0 |
| Sulphate | 1.60 | - | - | - |
| pH (1% solution) | 9.10 | 10.00 | 10.00 | 10.00 |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 16

The following liquid dishwashing detergent compositions were prepared in accord with the present invention :

| | **I** | **II** | **III** | **IV** | **V** |
|---|---|---|---|---|---|
| Alkyl (1-7) ethoxy sulfate | 28.5 | 27.4 | 19.2 | 34.1 | 34.1 |
| Amine oxide | 2.6 | 5.0 | 2.0 | 3.0 | 3.0 |
| C12 glucose amide | - | - | 6.0 | - | - |
| Betaine | 0.9 | - | - | 2.0 | 2.0 |
| Xylene sulfonate | 2.0 | 4.0 | - | 2.0 | - |
| Neodol C11E9 | - | - | 5.0 | - | - |
| Polyhydroxy fatty acid | - | - | - | 6.5 | 6.5 |
| amide | | | | | |
| Sodium diethylene penta | - | - | 0.03 | - | - |
| acetate (40%) | | | | | |
| Diethylenetriamine penta | - | - | - | 0.06 | 0.06 |
| acetate | | | | | |
| Sucrose | - | - | - | 1.5 | 1.5 |
| Ethanol | 4.0 | 5.5 | 5.5 | 9.1 | 9.1 |
| Alkyl diphenyl oxide | - | - | - | - | 2.3 |
| disulfonate | | | | | |
| Calcium formate | - | - | - | 0.5 | 1.1 |
| Ammonium citrate | 0.06 | 0.1 | - | - | - |
| Sodium chloride | - | 1.0 | - | - | - |
| Magnesium chloride | 3.3 | - | 0.7 | - | - |
| Calcium chloride | - | - | 0.4 | - | - |
| Sodium sulfate | - | - | 0.06 | - | - |
| Magnesium sulfate | 0.08 | - | - | - | - |
| Magnesium hydroxide | - | - | - | 2.2 | 2.2 |
| Sodium hydroxide | - | - | - | 1.1 | 1.1 |
| Hydrogen peroxide | 200ppm | 0.16 | 0.006 | - | - |
| Specific amylase enzyme | 0.005 | 0.003 | 0.01 | 0.008 | 0.008 |
| Complementary amylase | - | - | - | - | 0.008 |
| Protease | 0.5 | 0.15 | 0.10 | 0.08 | 0.05 |
| Perfume | 0.18 | 0.09 | 0.09 | 0.2 | 0.2 |
| Water and minors | Up to 100% | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 17

The following liquid hard surface cleaning compositions were prepared in accord with the present invention :

| | **I** | **II** | **III** | **IV** | **V** | **VI** | **VII** |
|---|---|---|---|---|---|---|---|
| Specific | 0.01 | 0.002 | 0.005 | 0.02 | 0.001 | 0.005 | 0.001 |
| amylase enzyme | | | | | | | |
| Complementary | - | - | - | - | - | - | .0005 |
| amylase | | | | | | | |
| Protease | 0.05 | 0.01 | 0.02 | 0.03 | 0.005 | 0.005 | 0.005 |
| EDTA* | - | - | 2.90 | 2.90 | - | - | - |
| Na Citrate | - | - | - | - | 2.90 | 2.90 | 2.90 |
| NaC12 Alkyl | 1.95 | - | 1.95 | - | 1.95 | - | - |
| benzene | | | | | | | |
| sulfonate | | | | | | | |
| NaC12 Alkyl | - | 2.20 | - | 2.20 | - | 2.20 | 2.20 |
| sulfate | | | | | | | |
| NaC12(ethoxy) | - | 2.20 | - | 2.20 | - | 2.20 | 2.20 |
| **sulfate | | | | | | | |
| C12 | - | 0.50 | - | 0.50 | - | 0.50 | 0.50 |
| Dimethylamine | | | | | | | |
| oxide | | | | | | | |
| Na Cumene | 1.30 | - | 1.30 | - | 1.30 | - | - |
| sulfonate | | | | | | | |
| Hexyl | 6.30 | 6.30 | 6.30 | 6.30 | 6.30 | 6.30 | 6.30 |
| Carbitol** | | | | | | | |
| Water | Up to 100% | | | | | | |

| | | | | | | | |
|---|---|---|---|---|---|---|---|
| *Na4 ethylenediamine diacetic acid | | | | | | | |
| **Diethylene glycol monohexyl ether ***All formulas adjusted to pH 7 | | | | | | | |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### Example 18

The following spray compositions for cleaning of hard surfaces and removing household mildew were prepared in accord with the present invention :

| | **I** | **II** |
|---|---|---|
| Specific Amylase Enzyme | 0.01 | 0.01 |
| Complementary amylase | - | 0.01 |
| Protease | 0.01 | 0.01 |
| Sodium octyl sulfate | 2.00 | 2.00 |
| Sodium dodecyl sulfate | 4.00 | 4.00 |
| Sodium hydroxide | 0.80 | 0.80 |
| Silicate (Na) | 0.04 | 0.04 |
| Perfume | 0.35 | 0.35 |
| Water/minors | up to 100% | up to 100% |

The same examples were reproduced with a specific amylase enzyme level of respectively 0.0004% and 0.04% pure enzyme by weight of total composition.

The level of the enzymes comprised in such composition are expressed in pure enzyme by weight of total composition.

### SEQUENCE LISTING

Sequence description : SEQ ID No. 1

Sequence description : SEQ ID No. 2

Sequence description : SEQ ID No. 3

Sequence description : SEQ ID No. 4

### SEQUENCE DESCRIPTION : SEQ ID No. 1

His His Asn Gly Thr Asn Gly Thr Met Met Gln Tyr Phe Glu Trp Tyr
1               5                   10                  15
Leu Pro Asn Asp Gly Asn His Trp Asn Arg Leu Arg Asp Asp Ala Ala
            20                  25                  30
Asn Leu Lys Ser Lys Gly Ile Thr Ala Val Trp Ile Pro Pro Ala Trp
        35                  40                  45
Lys Gly Thr Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Asn Gln Leu Gln Ala Ala Val Thr Ser Leu Lys Asn Asn Gly
                85                  90                  95
Ile Gln Val Tyr Gly Asp Val Val Met Asn His Lys Gly Gly Ala Asp
            100                 105                 110
Gly Thr Glu Ile Val Asn Ala Val Glu Val Asn Arg Ser Asn Arg Asn
        115                 120                 125
Gln Glu Thr Ser Gly Glu Tyr Ala Ile Glu Ala Trp Thr Lys Phe Asp
    130                 135                 140
Phe Pro Gly Arg Gly Asn Asn His Ser Ser Phe Lys Trp Arg Trp Tyr
145                 150                 155                 160
His Phe Asp Gly Thr Asp Trp Asp Gln Ser Arg Gln Leu Gln Asn Lys
                165                 170                 175
Ile Tyr Lys Phe Arg Gly Thr Gly Lys Ala Trp Asp Trp Glu Val Asp
            180                 185                 190
Thr Glu Asn Gly Asn Tyr Asp Tyr Leu Met Tyr Ala Asp Val Asp Met
        195                 200                 205
Asp His Pro Glu Val Ile His Glu Leu Arg Asn Trp Gly Val Trp Tyr
    210                 215                 220
Thr Asn Thr Leu Asn Leu Asp Gly Phe Arg Ile Asp Ala Val Lys His
225                 230                 235                 240
Ile Lys Tyr Ser Phe Thr Arg Asp Trp Leu Thr His Val Arg Asn Thr
                245                 250                 255
Thr Gly Lys Pro Met Phe Ala Val Ala Glu Phe Trp Lys Asn Asp Leu
            260                 265                 270

Gly Ala Ile Glu Asn Tyr Leu Asn Lys Thr Ser Trp Asn His Ser Val
        275                 280                 285
Phe Asp Val Pro Leu His Tyr Asn Leu Tyr Asn Ala Ser Asn Ser Gly
    290                 295                 300
Gly Tyr Tyr Asp Met Arg Asn Ile Leu Asn Gly Ser Val Val Gln Lys
305                 310                 315                 320
His Pro Thr His Ala Val Thr Phe Val Asp Asn His Asp Ser Gln Pro
                325                 330                 335
Gly Glu Ala Leu Glu Ser Phe Val Gln Gln Trp Phe Lys Pro Leu Ala
            340                 345                 350
Tyr Ala Leu Val leu Thr Arg Glu Gln Gly Tyr Pro Ser Val Phe Tyr
        355                 360                 365
Gly Asp Tyr Tyr Gly Ile Pro Thr His Gly Val Pro Ala Met Lys Ser
    370                 375                 380
Lys Ile Asp Pro leu Leu Gln Ala Arg Gln Thr Phe Ala Tyr Gly Thr
385                 390                 395                 400
Gln His Asp Tyr Phe Asp His His Asp Ile Ile Gly Trp Thr Arg Glu
                405                 410                 415
Gly Asn Ser Ser His Pro Asn Ser Gly Leu Ala Thr Ile Met Ser Asp
            420                 425                 430
Gly Pro Gly Gly Asn Lys Trp Met Tyr Val Gly Lys Asn Lys Ala Gly
        435                 440                 445
Gln Val Trp Arg Asp Ile Thr Gly Asn Arg Thr Gly Thr Val Thr Ile
    450                 455                 460
Asn Ala Asp Gly Trp Gly Asn Phe Ser Val Asn Gly Gly Ser Val Ser
465                 470                 475                 480
Val Trp Val Lys Gln
                485

### SEQUENCE DESCRIPTION : SEQ ID No. 2

His His Asn Gly Thr Asn Gly Thr Met Met Gln Tyr Phe Glu Trp His
1               5                   10                  15
Leu Pro Asn Asp Gly Asn His Trp Asn Arg Leu Arg Asp Asp Ala Ser
                20                  25                      30
Asn Leu Arg Asn Arg Gly Ile Thr Ala Ile Trp Ile Pro Pro Ala Trp
            35                  40                  45
Lys Gly Thr Ser Gln Asn Asp Val Gly Tyr Gly Ala Tyr Asp Leu Tyr
        50                  55                  60
Asp Leu Gly Glu Phe Asn Gln Lys Gly Thr Val Arg Thr Lys Tyr Gly
        65                  70                  75
Thr Arg Ser Gln Leu Glu Ser Ala Ile His Ala Leu Lys Asn Asn Gly
80                  85                  90                      95
Val Gln Val Tyr Gly Asp Val Val Met Asn His Lys Gly Gly Ala Asp
                100                 105                 110
Ala Thr Glu Asn Val Leu Ala Val Glu Val Asn Pro Asn Asn Arg Asn
            115                 120                     125
Gln Glu Ile Ser Gly Asp Tyr Thr Ile Glu Ala Trp Thr Lys Phe Asp
        130                 135                 140
Phe Pro Gly Arg Gly Asn Thr Tyr Ser Asp Phe Lys Trp Arg Trp Tyr
        145                 150                 155
His Phe Asp Gly Val Asp Trp Asp Gln Ser Arg Gln Phe Gln Asn Arg
160                 165                 170                     175
Ile Tyr Lys Phe Arg Gly Asp Gly Lys Ala Trl Asp Trp Glu Val Asp
                180                 185                 190
Ser Glu Asn Gly Asn Tyr Asp Tyr Leu Met Tyr Ala Asp Val Asp Met
            195                 200                 205
Asp His Pro Glu Val Val Asn Glu Leu Arg Arg Trp Gly Glu Trp Tyr
        210                 215                 220
Thr Asn Thr Leu Asn Leu Asp Gly Phe Arg Ile Asp Ala Val Lys His
    225                 230                 235
Ile Lys Tyr Ser Phe Thr Arg Asp Trp Leu Thr His Val Arg Asn Ala
240                 245                 250                     255
Thr Gly Lys Glu Met Phe Ala Val Ala Glu Phe Trp Lys Asn Asp Leu
                260                 265                 270

Gly Ala Leu Glu Asn Tyr Leu Asn Lys Thr Asn Trp Asn His Ser Val
275 280 285
Phe Asp Val Pro Leu His Tyr Asn Leu Tyr Asn Ala Ser Asn Ser Gly
290 295 300
Gly Asn Tyr Asp Met Ala Lys Leu Leu Asn Gly Thr Val Val Gln Lys
305 310 315
His Pro Met His Ala Val Thr Phe Val Asp Asn His Asp Ser Gln Pro
320 325 330 335
Gly Glu Ser Leu Glu Ser Phe Val Gln Glu Trp Phe Lys Pro Leu Ala
340 345 350
Tyr Ala Leu Ile Leu Thr Arg Glu Gln Gly Tyr Pro Ser Val Phe Tyr
355 360 365
Gly Asp Tyr Tyr Gly Ile Pro Thr His Ser Val Pro Ala Met Lys Ala
370 375 380
Lys Ile Asp Pro Ile Leu Glu Ala Arg Gln Asn Phe Ala Tyr Gly Thr
385 390 395
Gln His Asp Tyr Phe Asp His His Asn Ile Ile Gly Trp Thr Arg Glu
400 405 410 415
Gly Asn Thr Thr His Pro Asn Ser Gly Leu Ala Thr Ile Met Ser Asp
420 425 430
Gly Pro Gly Gly Glu Lys Trp Met Tyr Val Gly Gln Asn Lys Ala Gly
435 440 445
Gln Val Trp His Asp Ile Thr Gly Asn Lys Pro Gly Thr Val Thr Ile
450 455 460
Asn Ala Asp Gly Trp Ala Asn Phe Ser Val Asn Gly Gly Ser Val Ser
465 470 475
Ile Trp Val Lys Arg
480

### SEQUENCE DESCRIPTION : SEQ ID No. 3

His-His-Asn-Gly-Thr-Asn-Gly-Thr-Met-Met-Gln-Tyr-Phe-Glu-Trp-Tyr-Leu-Pro-
Asn-Asp

### SEQUENCE DESCRIPTION : SEQ ID No. 4

AAPFNGTMMQ YFEWYLPDDG TLWTKVANEA NNLSSLGITA LWLPPAYKGT
SRSDVGYGVY DLYDLGEFNQ KGAVRTKYGT KAQYLQAIQA AHAAGMQVYA
DVVFDHKGGA DGTEWVDAVE VNPSDRNQEI SGTYQIQAWT KFDFPGRGNT
YSSFKWRWYH FDGVDWDESR KLSRIYKFRG IGKAWDWEVD TENGNYDYLM
YADLDMDHPE VVTELKSWGK WYVNTTNIDG FRLDAVKHIK FSFFPDWLSD
VRSQTGKPLF TVGEYWSYDI NKLHNYIMKT NGTMSLFDAP LHNKFYTASK
SGGTFDMRTL MTNTLMKDQP TLAVTFVDNH DTEPGQALQS WVDPWFKPLA
YAFILTRQEG YPCVFYGDYY GIPQYNIPSL KSKIDPLLIA RRDYAYGTQH
DYLDHSDIIG WTREGVTEKP GSGLAALITD GPGGSKWMYV GKQHAGKVFY
DLTGNRSDTV TINSDGWGEF KVNGGSVSVW VPRKTTVSTI AWSITTRPWT
DEFVRWTEPR LVAWP

## Claims

1. A detergent composition comprising a protease and from 0.00018% to 0.06% pure enzyme by weight of total composition of :
(a) α-amylase **characterised by** having a specific activity at least 25% higher than the specific activity of Termamyl® at a temperature range of 25°C to 55°C and at a pH value in the range of 8 to 10, measured by the Phadebas® α-amylase activity assay and/or;
(b) α-amylase according (a) comprising the amino sequence shown in SEQ ID No. 1 or an α-amylase being at least 80% homologous with the amino acid sequence shown in SEQ ID No.1 and/or;
(c) α-amylase according (a) comprising the amino sequence shown in SEQ ID No.2 or an α-amylase being at least 80% homologous with the amino acid sequence shown in SEQ ID No.2 and/or;
(d) α-amylase according (a) comprising the following amino sequence in the N-terminal : His-His-Asn-Gly-Thr-Asn-Gly-Thr-Met-Met-Gln-Tyr-Phe-Glu-Trp-Tyr-Leu-Pro-Asn-Asp (SEQ ID No.3) or an α-amylase being at least 80% homologous with the amino acid sequence shown (SEQ ID No.3) in the N-terminal and/or;
(e) α-amylase according (a-d) wherein the α-amylase is obtainable from an alkalophilic Bacillus species and/or
(f) α-amylase according to (e) wherein the amylase is obtainable from any of the strains NCIB 12289, NCIB 12512, NCIB 12513 and DSM 935 and/or;
(g)α-amylase showing positive immunological cross-reactivity with antibodies raised against an α-amylase having an amino acid sequence corresponding respectively to SEQ ID No.1, ID No.2 or ID No.3 and/or;
(h) Variant of a parent α-amylase, which parent α-amylase (i) has one of the amino acid sequences shown in SEQ ID No.1, ID No.2 or ID No.4 respectively, or (ii)displays at least 80% homology with one or more of said amino acid sequences, and/or displays immunological cross-reactivity with an antibody raised against an α-amylase having one of said amino acid sequences, and/or is encoded by a DNA sequence wich hybridizes with the same probe as a DNA sequence encoding an α-amylase having one of said amino acid sequence; in which variants :
(i) at least one amino acid residue of said parent α-amylase has been deleted; and/or
(ii) at least one amino acid residue of said parent α-amylase has been replaced by a different amino acid residue; and/or
(iii) at least one amino acid residue has been inserted relative to said parent α-amylase;
said variant having an α-amylase activity and exhibiting at least one of the following properties relative to said parent α-amylase : increased thermostability, increased stability towards oxidation, reduced Ca ion dependency, increased stability and/or α-amylolytic activity at neutral to relatively high pH values, increased α-amylolytic activity at relatively high temperature and increase or decrease of the isoelectric point (pI) so as to better match the pI value for α-amylase variant to the pH of the medium.

2. A detergent composition according claim 1 wherein said α-amylase is comprised at a level from 0.00024% to 0.048% pure enzyme by weight of total composition.

3. A detergent composition according to any of the preceding claims further comprising a complementary amylase.

4. A detergent composition according to claim 3 wherein the complementary amylase is comprised at a level of from 0.0001% to 2%, preferably from 0.00018% to 0.06%, more preferably from 0.00024% to 0.048% pure enzyme by weight of the composition.

5. A detergent composition according to claims 3 to 4 wherein the weight ratio of specific amylase to a complementary amylase is comprised between 9:1 to 1:9, preferably between 4:1 to 1:4, more preferably between 2:1 and 1:2.

6. A detergent composition according to any of the preceding claims further comprising a dispersant.

7. A detergent composition according to any of the preceding claims further comprising a builder component.

8. A detergent composition according to claim 8 wherein said builder component is metasilicate.

9. A detergent composition according to any of the preceding claims further comprising a chlorine scavenger.

10. A detergent composition according to any of the preceding claims further comprising a bleach system.

11. A detergent composition according to any of the preceding claims further comprising one or more components selected from anionic, nonionic, cationic, amphoteric and zwitterionic surfactants, preferably a cationic surfactant.

12. A detergent composition according to any of the preceding claims, further comprising suds suppressors, soil suspension and anti-redeposition agents, smectite clays and the like.

13. A detergent composition according to any of the preceding claims **characterised in that** the composition is a granular detergent composition containing no more than 15% by weight of inorganic filler salt.

14. A detergent composition according to claims 1-8 **characterised in that** the composition is a heavy duty liquid composition.

15. A detergent composition according to any of the preceding claims further comprising other enzymes providing cleaning performance and/or fabric care benefits.

16. A detergent composition according to claim 1 which is in the form of a detergent additive.

17. Use of a detergent composition according to any of the preceding claims for hard surface cleaning and/or hand and machine dishwashing and/or laundry at a temperature from 10°C to 25°C.

## Patentansprüche

1. Detergenszusammensetzung, umfassend eine Protease und 0,00018% bis 0,06% reines Enzym, bezogen auf Gewicht der Gesamtzusammensetzung, an:
(a) α-Amylase, die **gekennzeichnet ist durch** eine spezifische Aktivität, die mindestens 25% höher ist als die spezifische Aktivität von Termamyl® bei einem Temperaturbereich von 25°C bis 55°C und bei einem pH-Wert im Bereich von 8 bis 10, gemessen **durch** den Phadebas® α-Amylase-Aktivitätsassay; und/oder
(b) α-Amylase gemäß (a), umfassend die in SEQ ID Nr. 1 gezeigte Aminosäuresequenz, oder eine α-Amylase, die zu mindestens 80% mit der in SEQ ID Nr. 1 gezeigten Aminosäuresequenz homolog ist; und/oder
(c) α-Amylase gemäß (a), umfassend die in SEQ ID Nr. 2 gezeigte Aminosäuresequenz oder eine α-Amylase, die zu mindestens 80% mit der in SEQ ID Nr. 2 gezeigten Aminosäuresequenz homolog ist; und/oder
(d) α-Amylase gemäß (a), umfassend die folgende Aminosäuresequenz am N-Ende: His-His-Asn-Gly-Thr-Asn-Gly-Thr-Met-Met-Gln-Tyr-Phe-Glu-Trp-Tyr-Leu-Pro-Asn-Asp (SEQ ID Nr. 3) oder eine α-Amylase, die mit der gezeigten Aminosäuresequenz (SEQ ID Nr. 3) am N-Ende zu mindestens 80% homolog ist; und/oder
(e) α-Amylase gemäß (a-d), wobei die α-Amylase erhältlich ist aus einer alkalophilen Bacillus Spezies; und/oder
(f) α-Amylase gemäß (e), wobei die Amylase erhältlich ist aus irgendeinem der Stämme NCIB 12289, NCIB 12512, NCIB 12513 und DSM 935; und/oder
(g) α-Amylase, die eine positive immunologische Kreuzreaktivität zeigt mit Antikörpern, die gegenüber einer α-Amylase mit einer Aminosäuresequenz korrespondierend zu SEQ ID Nr. 1, ID Nr. 2 oder ID Nr. 3 gebildet wurden; und/oder
(h) einer Variante einer Stamm-α-Amylase, welche Stamm-α-Amylase (i) eine der in SEQ ID Nr. 1, ID Nr. 2 oder ID Nr. 4 gezeigten Aminosäuresequenzen besitzt, oder (ii) mindestens 80% Homologie mit einer oder mehreren dieser Aminosäuresequenzen zeigt, und/oder immunologische Kreuzreaktivität mit einem Antikörper, der gegenüber einer α-Amylase mit einer dieser Aminosäuresequenzen gebildet wurde, zeigt und/oder **durch** eine DNA-Sequenz codiert wird, welche mit dem gleichen Fühler hybridisiert wie eine DNA-Sequenz, die eine α-Amylase codiert, die eine dieser Aminosäuresequenzen aufweist; wobei in diesen Varianten:
(i) mindestens ein Aminosäurerest der Stamm-α-Amylase deletiert worden ist; und/oder
(ii) mindestens ein Aminosäurerest der Stamm-α-Amylase **durch** einen verschiedenen Aminosäurerest ersetzt worden ist; und/oder
(iii) mindestens ein Aminosäurerest relativ zu der Stamm-α-Amylase insertiert worden ist;
wobei die Variante eine α-Amylaseaktivität besitzt und mindestens eine der folgenden Eigenschaften relativ zu der Stamm-α-Amylase zeigt: erhöhte Thermostabilität, erhöhte Stabilität gegenüber Oxidation, reduzierte Ca-Ionen-Abhängigkeit, erhöhte Stabilität und/oder α-amolytische Aktivität bei neutralen bis relativ hohen pH-Werten, erhöhte α-amolytische Aktivität bei relativ hohen Temperaturen und Zunahme oder Abnahme des isoelektrischen Punkts (pI), um so den pI-Wert für α-Amylasevariante besser an den pH des Mediums anzugleichen.

2. Detergenszusammensetzung nach Anspruch 1, wobei die α-Amylase in einem Anteil von 0,00024% bis 0.048% reines Enzym, bezogen auf Gewicht der Gesamtzusammensetzung, enthalten ist.

3. Detergenszusammensetzung nach mindestens einem der vorangehenden Ansprüche, umfassend weiterhin ein komplementäre Amylase.

4. Detergenszusammensetzung nach Anspruch 3, wobei die komplementäre Amylase in einem Anteil von 0,0001% bis 2%, vorzugsweise 0,00018% bis 0,06%, weiter vorzugsweise 0,00024% bis 0,048% reines Enzym, bezogen auf Gewicht der Zusammensetzung, enthalten ist.

5. Detergenszusammensetzung nach den Ansprüchen 3 bis 4, wobei das Gewichtsverhältnis der spezifischen Amylase zu einer komplementären Amylase zwischen 9:1 und 1:9, vorzugsweise zwischen 4:1 und 1:4, weiter vorzugsweise zwischen 2:1 und 1:2, liegt.

6. Detergenszusammensetzung nach mindestens einem der vorangehenden Ansprüche, umfassend weiterhin ein Dispergiermittel.

7. Detergenszusammensetzung nach mindestens einem der vorangehenden Ansprüche, umfassend weiterhin eine Builderkomponente.

8. Detergenszusammensetzung nach Anspruch 7, wobei die Builderkomponente Metasilicat ist.

9. Detergenszusammensetzung nach mindestens einem der vorangehenden Ansprüche, umfassend weiterhin einen Chlorfänger.

10. Detergenszusammensetzung nach mindestens einem der vorangehenden Ansprüche, umfassend weiterhin ein Bleichmittelsystem.

11. Detergenszusammensetzung nach mindestens einem der vorangehenden Ansprüche, umfassend weiterhin eine oder mehrere Komponenten, gewählt aus anionischen, nichtionischen, kationischen, amphoteren und zwitterionischen Tensiden, vorzugsweise ein kationisches Tensid.

12. Detergenszusammensetzung nach mindestens einem der vorangehenden Ansprüche, umfassend weiterhin Schaumunterdrücker, Schmutzsuspendier- und Antiwiederablagerungsmittel, Smectit-Tone und dergleichen.

13. Detergenszusammensetzung nach mindestens einem der vorangehenden Ansprüche, **dadurch gekennzeichnet, dass** die Zusammensetzung eine granuläre Detergenszusammensetzung ist, welche nicht mehr als 15 Gew.-% anorganischer Füllstoffsalze enthält.

14. Detergenszusammensetzung nach den Ansprüchen 1-8, **dadurch gekennzeichnet, dass** die Zusammensetzung eine Universal-Flüssigzusammensetzung ist.

15. Detergenszusammensetzung nach mindestens einem der vorangehenden Ansprüche, umfassend weiterhin andere Enzyme, welche Reinigungsleistungsund/oder Gewebepflegevorteile vorsehen.

16. Detergenszusammensetzung nach Anspruch 1, welche in Form eines Detergensadditivs vorliegt.

17. Verwendung einer Detergenszusammensetzung nach mindestens einem der vorangehenden Ansprüche zum Reinigen harter Oberflächen und/oder händischen und maschinellen Geschirrspülen und/oder Wäschewaschen bei einer Temperatur von 10°C bis 25°C.

## Revendications

1. Composition détergente comprenant une protéase et de 0,00018 % à 0,06 % d'enzyme pure, en poids de la composition totale, constituée de :
(a) une α-amylase **caractérisée en ce qu'**elle a une activité spécifique d'au moins 25 % supérieure à celle du Termamyl® à une température allant de 25 °C à 55 °C et à un pH compris dans la fourchette de 8 à 10, mesurée par le dosage d'activité d'α-amylase Phadebas® ; et/ou
(b) une α-amylase selon (a) comprenant la séquence d'acides aminés présentée dans SEQ ID N° 1 ou une α-amylase homologue à au moins 80 % à la séquence d'acides aminés présentée dans SEQ ID N° 1 ; et/ou
(c) une α-amylase selon (a) comprenant la séquence d'acides aminés présentée dans SEQ ID N° 2 ou une α-amylase homologue à au moins 80 % à la séquence d'acides aminés présentée dans SEQ ID N° 2 ; et/ou
(d) une α-amylase selon (a) comprenant la séquence d'acides aminés suivante dans la partie N-terminale : His-His-Asn-Gly-Thr-Asn-Gly-Thr-Met-Met-Gln-Tyr-Phe-Glu-Trp-Tyr-Leu-Pro-Asn-Asp (SEQ ID N° 3) ou une α-amylase homologue à au moins 80 % à la séquence d'acides aminés présentée (SEQ ID N° 3) dans la partie N-terminale ; et/ou
(e) une α-amylase selon (a-d) où l'α-amylase est susceptible d'être obtenue à partir d'une espèce de Bacillus alcalophile et/ou
(f) une α-amylase selon (e) où l'amylase est susceptible d'être obtenue à partir de l'une quelconque des souches NCIB 12289, NCIB 12512, NCIB 12513 et DSM 935 ; et/ou
(g) une α-amylase présentant une réaction croisée immunologique positive avec des anticorps dirigés contre une α-amylase ayant une séquence d'acides aminés correspondant respectivement à SEQ ID N° 1, ID N° 2 ou ID N° 3 ; et/ou
(h) un variant d'une α-amylase d'origine, laquelle α-amylase d'origine (i) possède l'une des séquences d'acides aminés présentées dans SEQ ID N° 1, ID N° 2 ou ID N° 4 respectivement, ou (ii) présente au moins 80 % d'homologie avec une ou plusieurs desdites séquences d'acides aminés, et/ou présente une réaction croisée immunologique avec un anticorps dirigé contre une α-amylase ayant l'une desdites séquences d'acides aminés, et/ou est codée par une séquence d'ADN qui s'hybride avec la même sonde qu'une séquence d'ADN codant pour une α-amylase ayant l'une desdites séquences d'acides aminés ; dans lesquels variants :
(i) au moins un résidu d'acide aminé de ladite α-amylase d'origine a été délété ; et/ou
(ii) au moins un résidu d'acide aminé de ladite α-amylase d'origine a été remplacé par un résidu d'acide aminé différent ; et/ou
(iii) au moins un résidu d'acide aminé a été inséré par rapport à ladite α-amylase d'origine ;
ledit variant ayant une activité α-amylase et présentant au moins l'une des propriétés suivantes par rapport à ladite α-amylase d'origine : une thermostabilité accrue, une stabilité accrue vis-à-vis de l'oxydation, une dépendance réduite aux ions Ca, une stabilité et/ou une activité α-amylolytique accrues à des valeurs de pH neutres à relativement élevées, une activité α-amylolytique accrue à une température relativement élevée et une augmentation ou une diminution du point isoélectrique (pI) pour mieux ajuster la valeur du pI du variant d'α-amylase par rapport au pH du milieu.

2. Composition détergente selon la revendication 1, dans laquelle ladite α-amylase est présente à raison de 0,00024 % à 0,048 % d'enzyme pure en poids de la composition totale.

3. Composition détergente selon l'une quelconque des revendications précédentes, comprenant en outre une amylase complémentaire.

4. Composition détergente selon la revendication 3, dans laquelle l'amylase complémentaire est présente à raison de 0,0001 % à 2 %, de préférence, 0,00018% à 0,06 %, plus préférablement 0,00024% à 0,048 % d'enzyme pure en poids de la composition.

5. Composition détergente selon les revendications 3 à 4, dans laquelle le rapport pondéral de l'amylase spécifique à une amylase complémentaire est compris entre 9:1 et 1:9, de préférence entre 4:1 et 1:4, plus préférablement entre 2:1 et 1:2.

6. Composition détergente selon l'une quelconque des revendications précédentes, comprenant en outre un dispersant.

7. Composition détergente selon l'une quelconque des revendications précédentes, comprenant en outre un composant adjuvant.

8. Composition détergente selon la revendication 7, dans laquelle ledit composant adjuvant est un métasilicate.

9. Composition détergente selon l'une quelconque des revendications précédentes, comprenant en outre un agent de piégeage du chlore.

10. Composition détergente selon l'une quelconque des revendications précédentes, comprenant en outre un système de blanchiment.

11. Composition détergente selon l'une quelconque des revendications précédentes, comprenant en outre un ou plusieurs composants choisis parmi les tensioactifs anioniques, non ioniques, cationiques, amphotères et zwittérioniques, de préférence un tensioactif cationique.

12. Composition détergente selon l'une quelconque des revendications précédentes, comprenant en outre des agents anti-mousse, des agents de mise en suspension et anti-redéposition des salissures, des argiles smectites et analogues.

13. Composition détergente selon l'une quelconque des revendications précédentes, **caractérisée en ce que** la composition est une composition détergente granulaire ne contenant pas plus de 15 % en poids de sel de charge inorganique.

14. Composition détergente selon l'une quelconque des revendications 1-8, **caractérisée en ce que** la composition est une composition liquide pour gros lavage.

15. Composition détergente selon l'une quelconque des revendications précédentes, comprenant en outre d'autres enzymes procurant des performances de nettoyage et/ou des avantages de soin des tissus.

16. Composition détergente selon la revendication 1, qui est sous forme d'un additif détergent.

17. Utilisation d'une composition détergente selon l'une quelconque des revendications précédentes pour le nettoyage des surfaces dures et/ou pour le lavage de la vaisselle à la main et en machine et/ou pour le lavage du linge à une température de 10 °C à 25 °C.
